# EUROPEAN PATENT SPECIFICATION

(11) **EP 3 775 286 B1**
(45) Date of publication and mention of the grant of the patent: **26.10.2022**
(21) Application number: 19729073.7
(22) Date of filing: 12.06.2019
(51) Int. Cl.: C12Q 1/6883, C12Q 1/6886

(54) **EPIGENETIC METHOD TO ASSESS SUN PROTECTION**
EPIGENETISCHES VERFAHREN ZUR BEURTEILUNG VOM SONNENSCHUTZ
PROCÉDÉ ÉPIGÉNÉTIQUE POUR ÉVALUER LA PROTECTION CONTRE LE SOLEIL

(30) Priority: 15.06.2018 EP 18177979
(43) Date of publication of application: 17.02.2021
(73) Proprietor: Unilever IP Holdings B.V., 3013 AL Rotterdam (NL); Unilever Global IP Limited, Wirral, Merseyside CH62 4ZD (GB)
(72) Inventor: DADD, Anthony, William, Sharnbrook Bedfordshire MK44 1LQ (GB); GUNN, David, Andrew, Sharnbrook Bedfordshire MK44 1LQ (GB); KAWATRA, Taniya, Surrey KT1 2BA (GB); NIP, John, Chun-Sing, Trumbull, Connecticut 06611 (US); ROCHA, Sheila, Alves, Trumbull, Connecticut 06611 (US)
(74) Representative: James, Helen Sarah
(86) International application number: PCT/EP2019/065416
(87) International publication number: WO 2019/238793

(56) References cited:
- WO-A1-2017/165199
- AMY R VANDIVER ET AL: "Age and sun exposure-related widespread genomic blocks of hypomethylation in nonmalignant skin", GENOME BIOLOGY, BIOMED CENTRAL LTD., LONDON, GB, vol. 16, no. 1, 16 April 2015 (2015-04-16) , page 80, XP021221763, ISSN: 1465-6906, DOI: 10.1186/S13059-015-0644-Y cited in the application
- CAMPUZANO-GARCIA A E: "DNA methylation in malar melasma and its improvement by sunscreen, retinoic acid and niacinamide", JOURNAL OF INVESTIGATIVE DERMATOLOGY 20180501 ELSEVIER B.V. NLD, vol. 138, no. 5, Supplement 1, 1 May 2018 (2018-05-01), XP009507064, ISSN: 1523-1747
- YAO SHEN ET AL: "Epigenetic and genetic dissections of UV-induced global gene dysregulation in skin cells through multi-omics analyses", SCIENTIFIC REPORTS, vol. 7, no. 1, 17 February 2017 (2017-02-17), XP055495308, DOI: 10.1038/srep42646

## Description

### TECHNICAL FIELD OF INVENTION

The present invention relates to an epigenetic method for obtaining information useful to determine the effect of the use of sun protection products on the skin of an individual.

### BACKGROUND TO INVENTION

DNA methylation is an epigenetic determinant of gene expression. Patterns of CpG methylation are heritable, tissue specific, and correlate with gene expression. The consequence of methylation, particularly if located in a gene promoter, is usually gene silencing. DNA methylation also correlates with other cellular processes including embryonic development, chromatin structure, genomic imprinting, somatic X-chromosome inactivation in females, inhibition of transcription and transposition of foreign DNA and timing of DNA replication. When a gene is highly methylated it is less likely to be expressed. Thus, the identification of sites in the genome containing 5-meC is important in understanding cell-type specific programs of gene expression and how gene expression profiles are altered during both normal development, ageing and diseases such as cancer. Mapping of DNA methylation patterns is important for understanding diverse biological processes such as the regulation of imprinted genes, X chromosome inactivation, and tumour suppressor gene silencing in human cancers.

Ultraviolet (UV) irradiation present in sunlight is an environmental human carcinogen. The toxic effects of UV from natural sunlight and therapeutic artificial lamps are a major concern for human health. The major acute effects of UV irradiation on normal human skin comprise sunburn inflammation erythema, DNA damage, tanning, and local or systemic immunosuppression.

Sun protection products are topically applied skin treatments or oral supplements or foods capable of preventing or reducing UV rays reaching the skin and are therefore used to prevent or reduce the effect of exposure of the skin to sun.

It would be a highly valuable progression to be able to assess the epigenetic impact of the use of sun protection products in order to assess damage or health and to use that information to provide sun protection advice and interventions based on the epigenetic response of an individual to such products.

Various studies have investigated epigenetic changes in skin (i.e. DNA methylation) associated with sun exposure. Raddatz et al (Epigenetics & Chromatin 2013, 6:36) found that the aging epidermis methylome is characterized by considerable stability and does not show any global methylation loss. However, local age-related methylation changes could be observed, and were enriched at weak or poised promoters and at enhancer regions, thus suggesting a functional relevance of epigenetic mechanisms for skin aging. Gronniger et al. (PLoS Genetics 6(5): e1000971) found that aging and sun exposure are associated with comparably small, but significant changes in the DNA methylation patterns of human epidermis and dermis samples.

However, neither study investigated the epigenetic effect of the use of sun protection products. There therefore remains the need to provide an epigenetic method to assess the use of sun protection products, assess the sun protection requirements of the individual and their response to such products, and potentially use that information to provide advice and interventions personalised to the epigenetic sun protection profile of an individual.

WO 2017/165199 relates to a method for obtaining information useful to determine the effect of the use of sun protection products on sun exposure on the skin of an individual. The method comprises expression analysis of UVR-responsive biomarker genes, rather than methylation analysis.

### SUMMARY OF INVENTION

The invention is defined by the scope of the appended claims.

The inventors have surprisingly found a specific set of CpG sites that have enhanced efficacy in determining the effect of use of sun protection products on an individual and that can be used in an epigenetic method to assess the impact of the use of sun protection products. Therefore, the present invention provides a method for obtaining information useful to determine the effect of the use of sun protection products on chronic sun exposure on the skin of an individual, the method comprising the steps of:
(a) obtaining genomic DNA from skin cells derived from the individual, wherein the skin cells include epidermal skin cells; and
(b) observing cytosine methylation of at least two CpG loci in the genomic DNA selected from the group consisting of:
   cg02066936 cg16929393 cg06427571,
   so that information useful to determine the effect of the use of sun protection products on chronic sun exposure is obtained.

Preferably 2, most preferably all 3 sites of this subset are observed.

The invention also provides a method for obtaining information useful to determine the effect of the use of sun protection products on acute sun exposure on the skin of an individual, the method comprising the steps of:
(a) obtaining genomic DNA from skin cells derived from the individual, wherein the skin cells include epidermal skin cells; and
(b) observing cytosine methylation of at least two CpG loci in the genomic DNA selected from the group consisting of: 
cg22821554 cg08781365 cg09914773 cg07166800 cg22834542 cg04935109
cg08903441 cg04424885 cg03819134 cg13366863 cg13852284 cg03985520
cg12577010 cg18156845 cg07982208 cg12907959 cg06769820 cg08267250
cg23224396 cg13896748 cg02152631 cg19009417 cg05888872 cg04659582
cg06427571 cg21855021 cg17206555 cg10494639 cg16777510 cg16293892
cg24349610 cg09407650 cg27181375 cg02261047 cg23225508 cg17117459
cg22923895 cg10113820 cg13213009 cg02491003 cg16808156 cg00772091
cg17341765 cg02018681 cg01823161 cg06066180 cg26166717 cg15674181
cg00297912 cg19145082 cg23909173 cg15701237 cg02754084 cg15084618
cg00415971 cg25034941 cg18833788 cg17180088 cg02215357 cg05355684
cg05799507 cg08584947 cg06650378 cg26063719 cg10634273 cg24591969
cg26837962 cg16929393 cg12474705 cg10481072 cg23976431 cg00953443
cg23301462 cg23036668 cg07695089 cg20419272 cg24490133 cg04258138
cg24534872 cg19990373 cg01797450 cg01166892 cg08703857 cg15059474
cg09657114 cg10294853 cg27305903 cg13582226 cg14076239 cg27005906
cg19720260 cg05715751 cg13765278 cg10267724 cg20187049 cg19263548
cg05751344 cg02768671 cg25204319,
 so that information useful to determine the effect of the use of sun protection products on acute sun exposure is obtained.

Preferably >5, >10, >15, >20, >30, >50, >75, >90, most preferably all 99 sites of this subset are observed.

This method preferably involves observing cytosine methylation of at least two CpG loci in the genomic DNA selected from the group consisting of: 
cg22821554 cg08781365 cg09914773 cg07166800 cg22834542 cg04935109
cg08903441 cg04424885 cg03819134 cg13366863 cg13852284 cg03985520
cg12577010 cg18156845 cg07982208 cg12907959 cg06769820 cg08267250
cg23224396 cg13896748 cg02152631 cg19009417 cg05888872 cg04659582
cg06427571 cg21855021 cg17206555 cg10494639 cg16777510 cg16293892
cg24349610 cg09407650 cg27181375 cg02261047 cg23225508 cg17117459
cg22923895 cg10113820 cg13213009 cg02491003 cg16808156 cg00772091
cg17341765 cg02018681 cg01823161 cg06066180 cg26166717 cg15674181
cg00297912 cg19145082 cg23909173 cg15701237 cg02754084 cg15084618
cg00415971 cg25034941 cg18833788 cg17180088 cg02215357 cg05355684
cg05799507 cg08584947 cg06650378 cg26063719 cg10634273 cg24591969
cg26837962 cg16929393 cg12474705 cg10481072 cg01166892 cg08703857
cg15059474 cg09657114 cg10294853 cg27305903 cg13582226 cg14076239
cg27005906 cg19720260 cg05715751,
 so that information useful to determine the effect of the use of sun protection products on acute sun exposure is obtained.

Preferably >5, >10, >15, >20, >30, >50, >75, most preferably all 81 sites of this subset are observed.

This method most preferably involves observing cytosine methylation of at least two CpG loci in the genomic DNA selected from the group consisting of: 
cg22821554 cg08781365 cg09914773 cg07166800 cg22834542 cg04935109
cg08903441 cg04424885 cg03819134 cg13366863 cg13852284 cg03985520
cg12577010 cg18156845 cg07982208 cg12907959 cg06769820 cg08267250
cg23224396 cg13896748 cg02152631 cg19009417 cg05888872 cg04659582
cg06427571 cg21855021 cg17206555 cg10494639 cg16777510 cg16293892
cg24349610 cg09407650 cg27181375 cg02261047 cg23225508 cg01166892
cg08703857 cg15059474,
 so that information useful to determine the effect of the use of sun protection products on acute sun exposure is obtained.

Preferably >5, >10, >15, >20, >30, >35, most preferably all 38 sites of this subset are observed.

### DRAWINGS

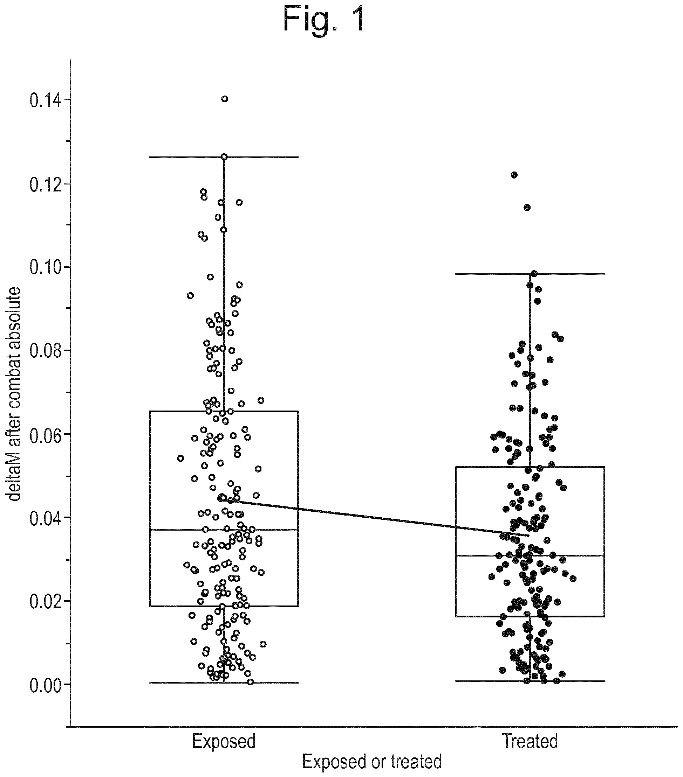

Figure 1 shows a boxplot of mean change in absolute M-value over 5 months for each of the 185 probes by treatment group. The figure shows the change in M-value measure (mean absolute change in M-value from baseline over samples) for each of the 185 probes in the treated and untreated groups separately. Values for individual probes are shown as points. Jitter was applied to individual points which accounts for the horizontal spread. Treated samples are shown on the left, untreated are shown on the right. The box plot middle line represents the median value of the 185, the line joining the two arms is joining the mean values.

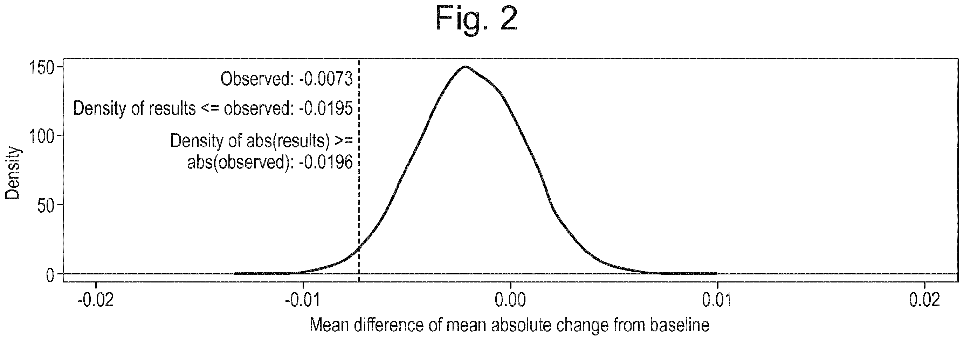

Figure 2 shows the Permutation analysis results. The figure shows the results of 10,000 permutations to calculate the mean change in M-value measure for sets of randomly sampled 185 probes. The point estimate for the selected 185 probes is shown as a dashed vertical line. The mean difference in mean absolute change from baseline was significantly different to the permuted (null) distribution (p = 0.0196).

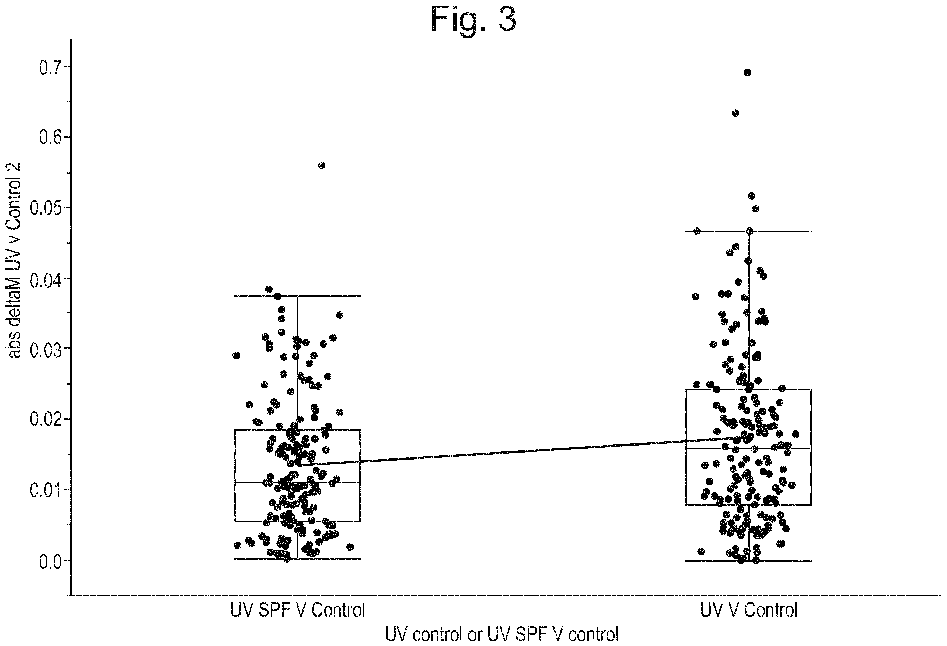

Figure 3 shows a Boxplot of mean change in absolute M-value over 1 week for each of the 185 probes by treatment group (SPF vs. no-SPF). The figure shows the change in M-value measure (mean absolute change in M-value from baseline over samples) for each of the 185 probes in the "UV vs. Control" changes (right hand box plot) and the "UV+SPF vs. Control" (left hand box plot) changes separately. Values for individual probes are shown as points. Jitter was applied to individual points which accounts for the horizontal spread. The box plot middle line represents the median value of the 185, the line joining the two arms is joining the mean values.

### DETAILED DESCRIPTION OF INVENTION AND EXAMPLES

Ultraviolet (UV) irradiation present in sunlight is an environmental human carcinogen. The toxic effects of UV from natural sunlight and therapeutic artificial lamps are a major concern for human health. The major acute effects of UV irradiation on normal human skin comprise sunburn inflammation erythema, tanning, and local or systemic immunosuppression. Malignant melanoma can be caused by indirect DNA damage from UVA radiation. In addition, other wavelengths of sunlight have also been linked to skin ageing (e.g. blue light and infrared light). As used herein the terms "sun exposure", "exposure", "exposure to sun", "UV exposure", and the like are intended to refer to exposure to either natural sunlight or artificial exposure such as sun beds and sun lamps. The term therefore refers to exposure to the harmful radiation associated with such, including but not limited to UVA and UVB radiation.

Sun exposure can occur over varying time scales. As used herein the term chronic sun exposure means exposure over the period of from 1 to 24 months, in particular about 5 months. As used herein the term acute sun exposure means exposure over the period of from 1 to 30 days, in particular about a week.

In the context of the present invention, sun protection products means topically applied skin treatments capable of preventing or reducing UV rays reaching the skin. Sun protection products may be referred to by terms such as sunscreen, sunblock, sun cream, or suntan lotion. They may be in the form of a lotion, spray, gel or other topical product. They may act by absorbing or reflecting some or all of the ultraviolet radiation from the sun, in particular UVA and/or UVB and therefore protect against sun exposure. Sun protection products often have an SPF rating (sun protection factor) which is a measure of the fraction of sunburn-producing UV rays that reach the skin.

As used herein, CpG sites/loci refer to the unique identifiers found in the Illumina CpG loci database (as described in Technical Note: Epigenetics, CpG Loci Identification ILLUMINA Inc. 2010, https://www.illumina.com/documents/products/technotes/technote_cpg_loci_identification.pdf). These CpG site identifiers therefore provide consistent and deterministic CpG loci database to ensure uniformity in the reporting of methylation data.

### Identification of CpG sites potentially associated with use of sun protection products

A study was carried out to identify CpG sites potentially associated with protection from sun exposure though the use of sun protection products.

Data from a single centre, cross-sectional biopsy study involving 24 Chinese and 24 European female participants in which 24 young and 24 old females had enrolled was used to identify CpG sites with potential value for the assessment of sun protection. Biopsy samples of skin were collected from two different areas of each subject: samples from a sun exposed area of the skin; and samples from a sun protected area of the skin. Areas of the skin designated as sun exposed were located on the lower outer arm and areas designated as sun protected were located on the upper inner arm, typically half way between the elbow and axilla area.

DNA methylation data from samples collected from exposed areas of skin in the young European subjects was compared with data from protected areas in the same young European subjects. After p-value filter and multiple testing correction (<0.05 FDR and 5% delta beta) 4450 CpG sites were found to be significantly different in the sun exposed samples relative to the sun protected samples and are therefore likely to be associated with sun protection.

Methylation data from samples collected from exposed skin areas in the young European subjects was compared with data from exposed skin areas in the old European subjects. After p-value filter and multiple testing correction (<0.05 False Discover Rate (FDR) and 5% delta beta) 6972 CpG sites were found to be significantly different in the old exposed samples relative to the young exposed samples and are therefore likely to be associated with prolonged sun exposure.

It was found that there was an overlap of 455 CpG sites between these two lists (i.e. between the 4450 and the 6972 CpG sites) and that of these 455 CpG sites, 399 were in the same direction - i.e.
- a CpG site from a young exposed sample was found to be hyper-methylated compared to the same CpG site from a young protected sample (i.e. hyper-methylation was caused by exposure)
and
- a CpG site from an old exposed sample was found to be hyper-methylated compared to a CpG site from a young exposed sample (i.e. hyper-methylation was caused by age)
or, conversely:
- a CpG site from a young exposed sample was found to be hypo-methylated compared to the same CpG site from a young protected sample (i.e. hypo-methylation was caused by exposure)
and
- a CpG site from an old exposed sample was found to be hypo-methylated compared to a CpG site from a young exposed sample (i.e. hypo-methylation was caused by age).

These 399 CpG sites found from the above single centre, cross-sectional biopsy study were then reassessed in a publicly available dataset (Vandiver A.R. et al.: Age and sun exposure-related widespread genomic blocks of hypomethylation in nonmalignant skin. Genome Biology (2015) 16:80, Gene Expression Omnibus accession number: GSE51954). The Vandiver dataset contained epidermal samples (N=38) from 20 Caucasian subjects. Paired punch biopsy samples, 4 mm in diameter, had been collected under local anaesthesia from sun exposed areas (outer forearm or lateral epicanthus) and sun protected areas (upper inner arm).

Of the 399 CpG sites identified from this study, 198 CpG sites were found to be significantly different (FDR for 399 tests, p<0.05, delta beta greater than 5%) comparing young to old exposed sites, and also significant (FDR for 399 tests, p<0.05, delta beta greater than 5%) comparing exposed to protected (separately tested) in the young samples from the Vandiver dataset.

Moreover, all 198 of these CpG sites were found to change in the same direction in both the Vandiver dataset and the above single centre, cross-sectional biopsy study (135 being hypo-methylated and 63 being hyper-methylated in the older photo-exposed samples).

These 198 CpG sites were therefore taken forward and assessed for their ability to measure the effect of using sun protection products in general and specifically the effect of using sun protection products during chronic and acute sun exposure.

### Identification of CpG sites associated with use of sun protection products on chronic sun exposure

A clinical study was carried out to investigate the epigenetic effect of using sun protection products on chronic sun exposure.

One aim of the study was to assess of the ability of the 198 CpG sites identified above to measure the effect of the use of a sun protection product on chronic sun exposure.

In the study, skin biopsy samples were collected from both forearms at the start of the study. The study then ran for 5 summer months during which a sun protection product (Pond's Age Miracle Firm and Lift Day Cream, Sun Protection Factor 30) was applied to one of the forearms ("treated") but not the other ("untreated"). At the end of the 5 months, skin biopsy samples were collected from both forearms to assess the effect of chronic sun exposure on the skin and to also assess the effect of using and not using the sun protection product for those 5 months.

The study was a single-centre, split forearm study with 42 subjects completing. Subjects were female, age 22-58, Fitzpatrick skin type II-III, in general good health, not regular users of sun protection (on the arms) and routinely went outside and were exposed to the sun (at least 10 hours per week).

Subjects used their typical facial and body cleansing and care products and routines and did not change their products and routine until the study completed.

Applications of the sun protection product were conducted daily, at home, by the subjects. Subjects were instructed to apply the product at least twice daily (subjects could apply morning, prior to going outside, every 2 hours while outside, or evening) to a randomly selected (right or left) forearm (consistent throughout study). Subjects were allowed to apply as many times a day as desired and when they went out in the sun. Subjects kept a diary on their sun exposure and sun protection product usage for 5 months.

At baseline and at month 5, 3-mm punch biopsies (1 from each extensor forearm at each time point) were collected by a licensed certified dermatologist. Resulting in the following biopsies
- Time 0 - free life-style forearm (untreated)
- Time 0 - forearm on which the test product was to also be applied (treated)
- Time 5 months - free life style forearm after 5 months (untreated)
- Time 5 months - forearm on which the test product had been applied over the 5 months (treated).

Baseline and month 5 biopsy sites were randomized within the forearm, either at upper (close to elbow), or lower (close to wrist) location. The two biopsy sites were about 4 - 5 cm apart.

A total of 168 biopsies were collected (42 subjects x 2 forearms per subject x 2 time points per forearm). Biopsies were analysed for epigenetic modifications caused by sun exposure on Illumina Infinium MethylationEPIC BeadChip arrays.

### EPIGENETIC ANALYSIS

One subject was missing one of the four sampling points (due to low DNA quantity), and as a result all observations for this subject were excluded from the analysis resulting in 41 individuals being available for further analysis (total 164 biopsies).

Of the 198 CpG sites identified above that were taken forward and assessed in the context of sun exposure and sun protection, 185 sites were present on the MethylationEPIC BeadChip arrays used.

Samples were filtered to remove probes that contained known polymorphisms (23,356 probes), were cross-hybridised (42,529 probes) and non-CpG probes (1,652 probes) identified by McCartney et al (Genomics Data, Volume 9, September 2016, Pages 22-24). Those probes on X and Y chromosomes (14,404 probes) and those probes where more than 25% of samples had a signal indistinguishable from negative control probes were also removed (7,094 probes). 83,801 probes were therefore excluded from the original 866,836, leaving 782,437 probes. None of the above 185 sites/probes were lost as a result of this filtering. The resultant dataset was corrected using ComBat to control for a batch effect in the data (related to the technical factor "Sentrix Barcode").

The overall aim of the study was to identify differentially methylated sites between baseline and the end of the study for both treated and untreated arms. Following this, the differences in post vs pre M values (paired delta-M values) were calculated for the treated and untreated arms. Delta-M measures for each probe were then modelled as a function of baseline M-value (a covariate), and subject and treatment group (fixed effects) using ordinary least squares. Specific contrasts of interest were then assessed by determining least-square means (or estimated marginal means) for delta-M (being different from 0); these contrasts were for
1. treated arms,
2. untreated arms, and
3. the difference between treated and untreated arms.

An extensive analysis of the 185 CpG sites of interest was carried out. Random sampling and permutation testing was performed to determine the performance of these 185 sites.

### RESULTS

For each probe and treatment group, the mean absolute change in M-value (from baseline) was calculated over samples. It was then determined whether this measure changed between untreated and treated samples over the set of probes.

To visualise changes for each of the 185 probes (averaged over samples within a treatment group), box plots (Figure 1) were generated for the untreated and treated groups separately. Each point in the boxplot shows the mean M-value measure (mean absolute change in M-value from baseline over samples) for a given probe and treatment group.

To test changes in M-value measures between untreated and treated samples the point estimate of change in M-value measure for the 185 probes between groups was first calculated. The significance of this estimate was tested using permutation testing in which 10,000 permutations of the procedure were performed for randomly chosen sets of 185 probes to construct the null distribution of change in M-value measure. It was then tested whether our point estimate was significantly different to this null distribution (Figure 2).

Permutation testing therefore demonstrates an observed value of -0.0073, indicating that the mean difference in mean absolute change from baseline across 185 probes was 0.0073 higher in the untreated group relative to the treated group. This mean difference in mean absolute change from baseline was significantly different to the permuted (null) distribution (p < 0.05).

Although individual probes showed mixed patterns of change in M-value measure between groups (with both negative and positive changes), when averaging over 185 probes, it was found that the observed mean change was significantly different to that observed for randomly chosen probes.

It is remarkable that methylation changes were observed in this specifically selected (predefined) probe set. Moreover, it is a novel insight that epigenetic changes were observed over just 5 months - typically such changes have been associated with many years of exposure.

It is therefore apparent that selections of these 185 CpG sites, are capable of measuring changes associated with or caused by the use of sun protection products and are therefore used in the method according to claim 1.

These sites and the more preferred selections of the sites listed below may also be used in a kit for obtaining information useful to assess changes associated with or caused by the use of sun protection products.

Further disclosed is a kit comprising primers or probes specific for at least two genomic DNA sequences in a biological sample, wherein the genomic DNA sequences comprise CpG loci in the genomic DNA selected from a group consisting only of the sites defined in claim 1 or claim 2;
and
a reagent used in:
a genomic DNA polymerization process;
a genomic DNA hybridization process;
a genomic DNA direct sequencing process;
a genomic DNA bisulphite conversion process; or
a genomic DNA pyrosequencing process.

The kit may comprise primers or probes specific for at least two genomic DNA sequences in a biological sample, wherein the genomic DNA sequences comprise CpG loci in the genomic DNA selected from a group consisting of any of the preferred sites defined in claim 3 or claim 4.

The 185 sites are shown in Table 1 in which annotations are based on Human Genome Build HG19 and the columns are:
- CpG site/loci ID
- Chromosome
- Probe start position
- Closest gene name
- Expected change in CpG site over time (where '+' means hyper-methylated, '-' means hypo- methylated)
- Whether CpG site was found to change in the expected direction (Yes/No)
- Whether CpG site was found to change in the expected direction AND showed greater change in the untreated arm than the treated arm (Yes/No)
- deltaM untreated (change in M-values of untreated arm)
- deltaM treated (change in M-values of treated arm)
- Difference in deltaM between untreated and treated arms
- P-value for change in untreated arm
- P-value for change in untreated vs. treated arm

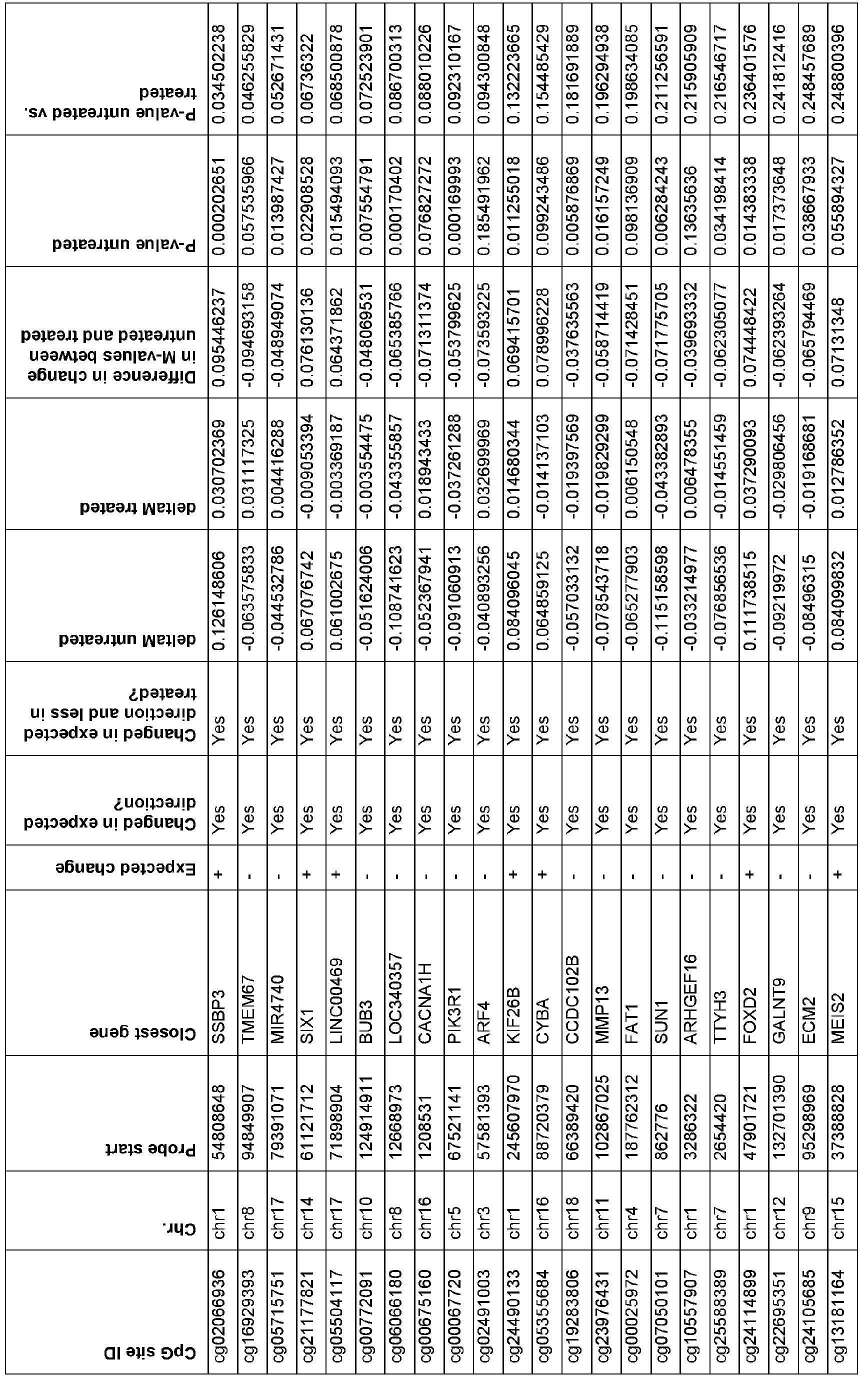

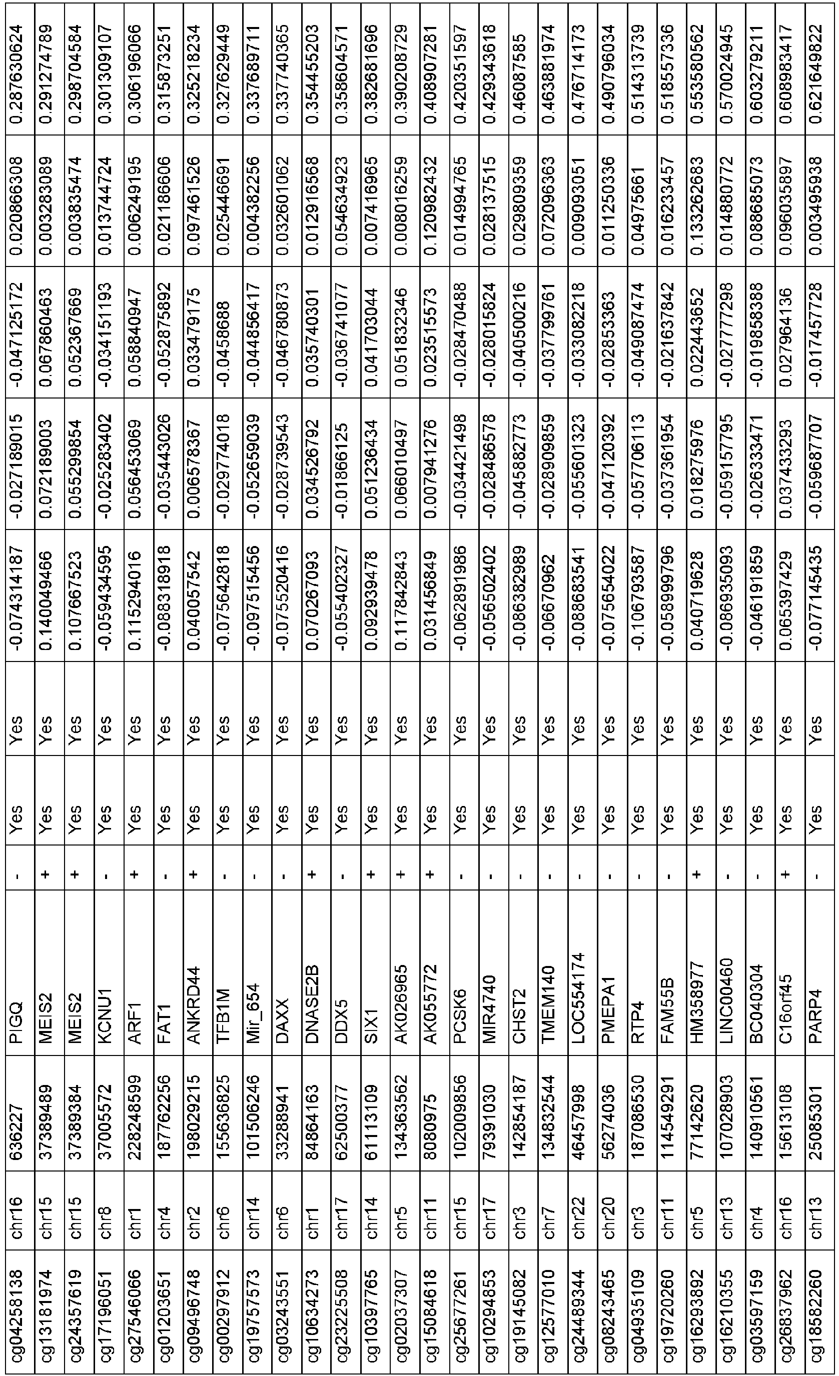

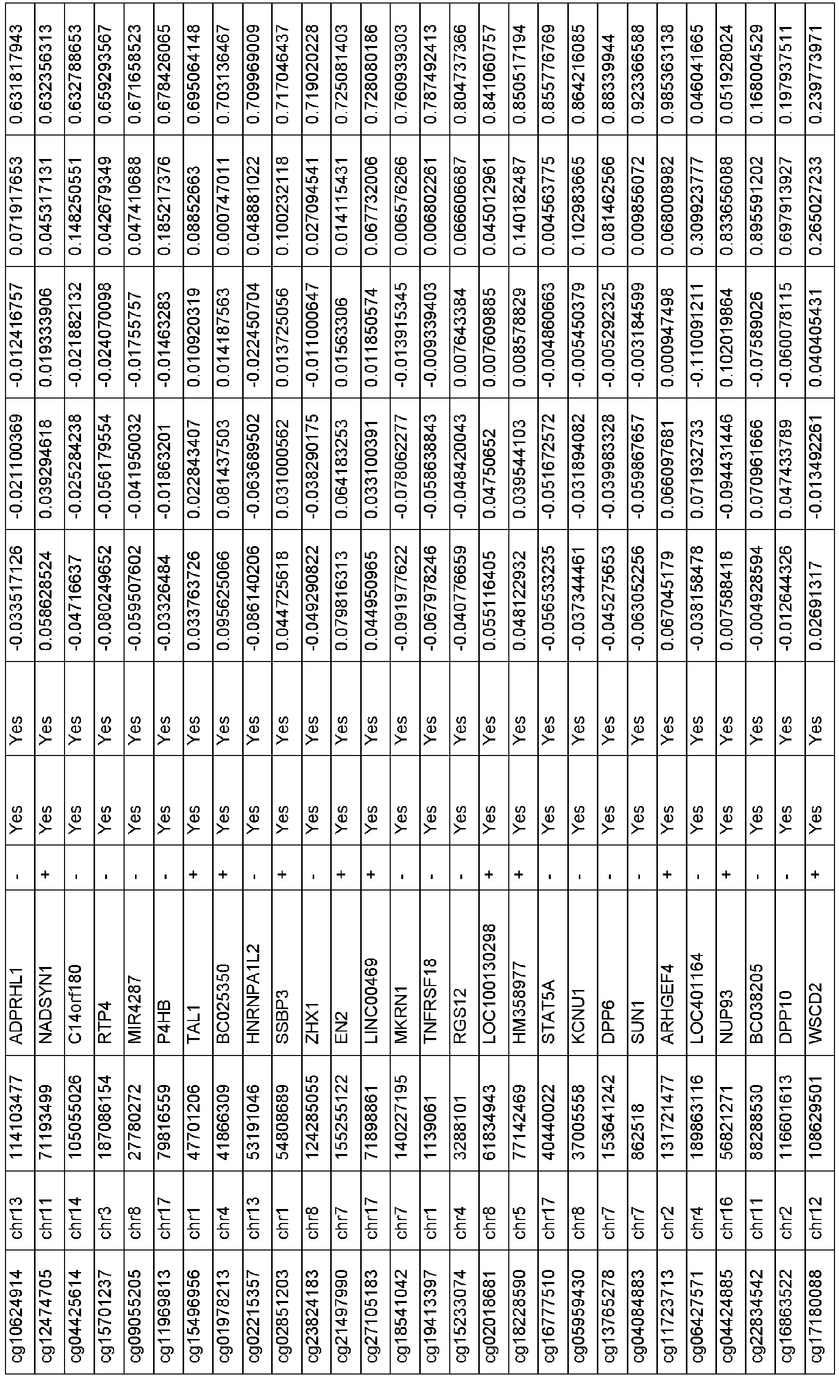

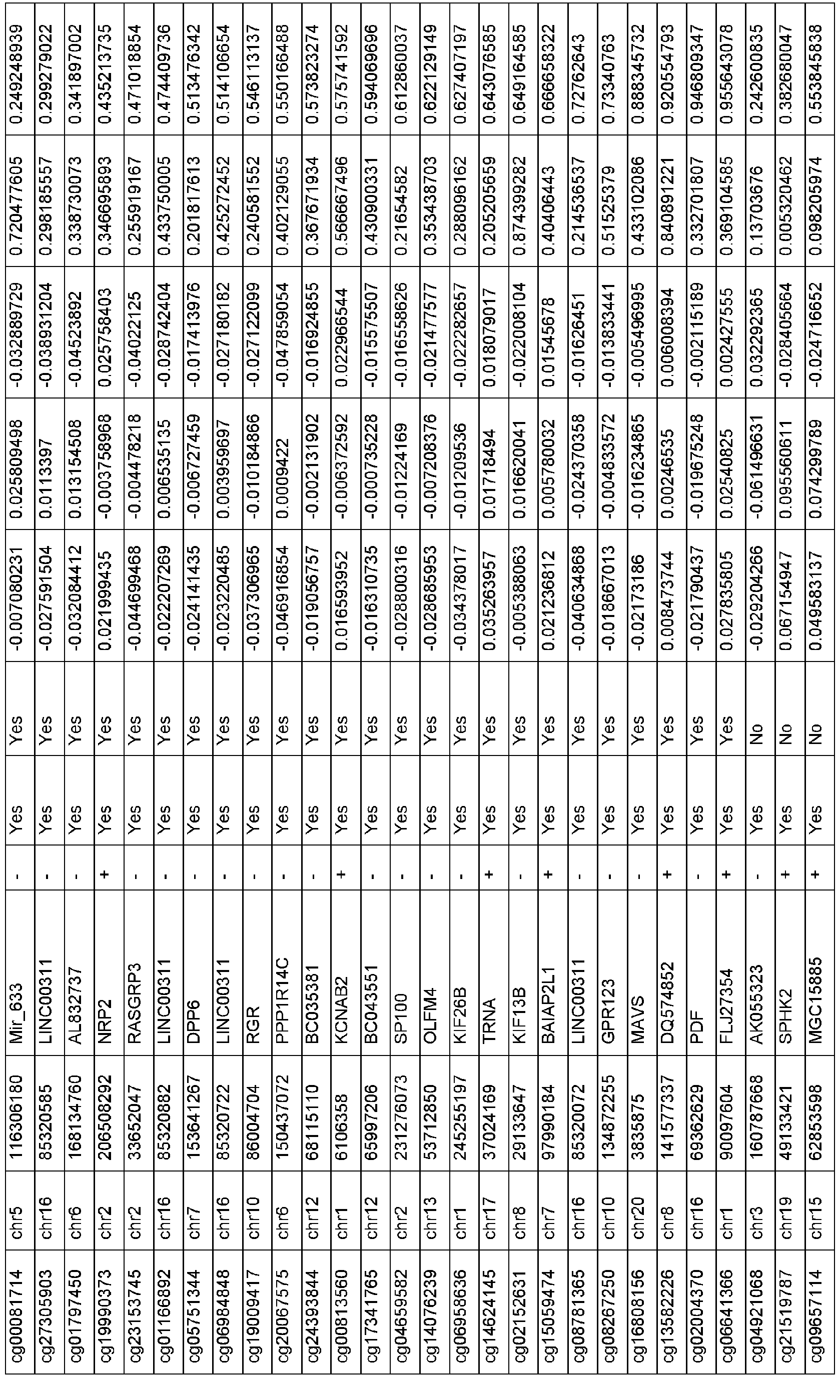

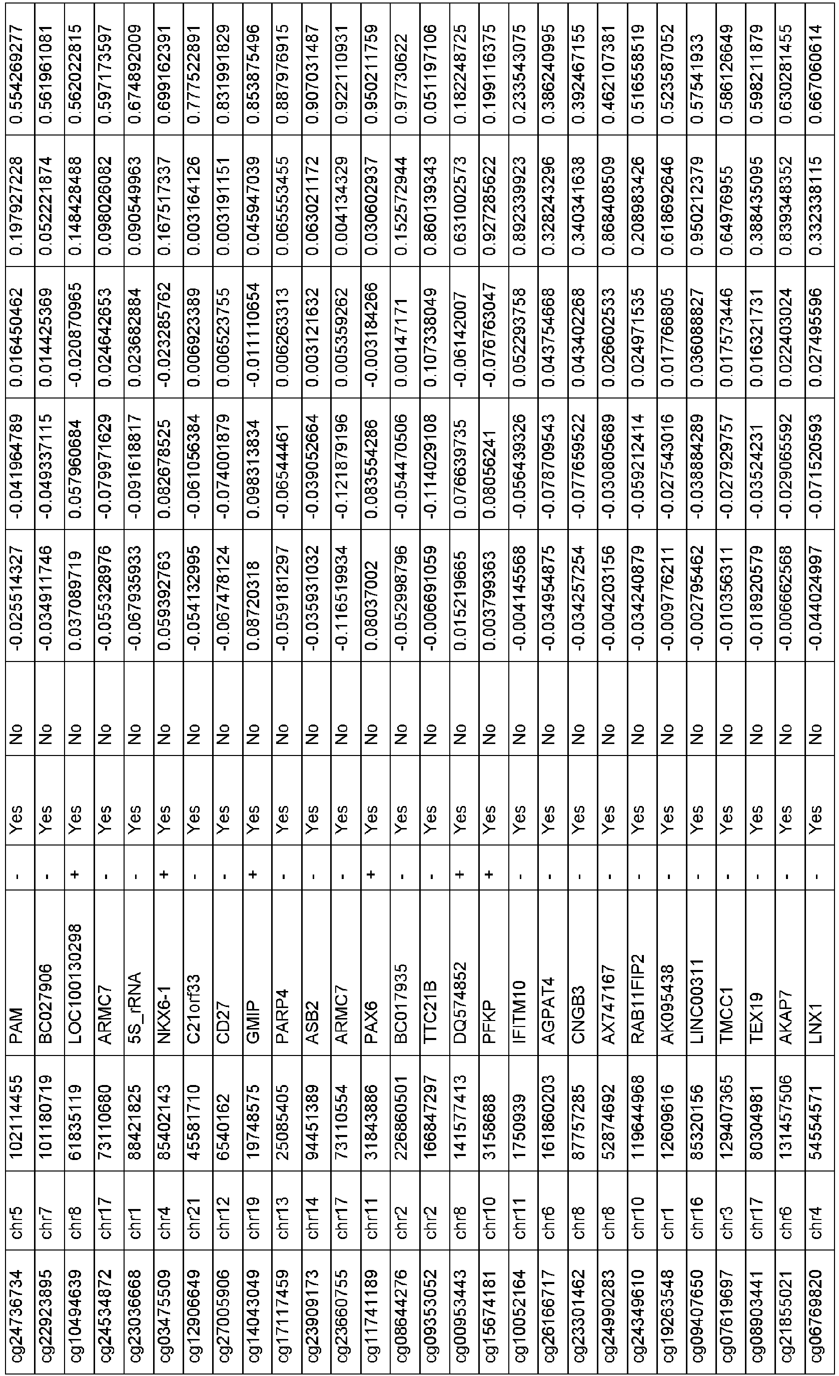

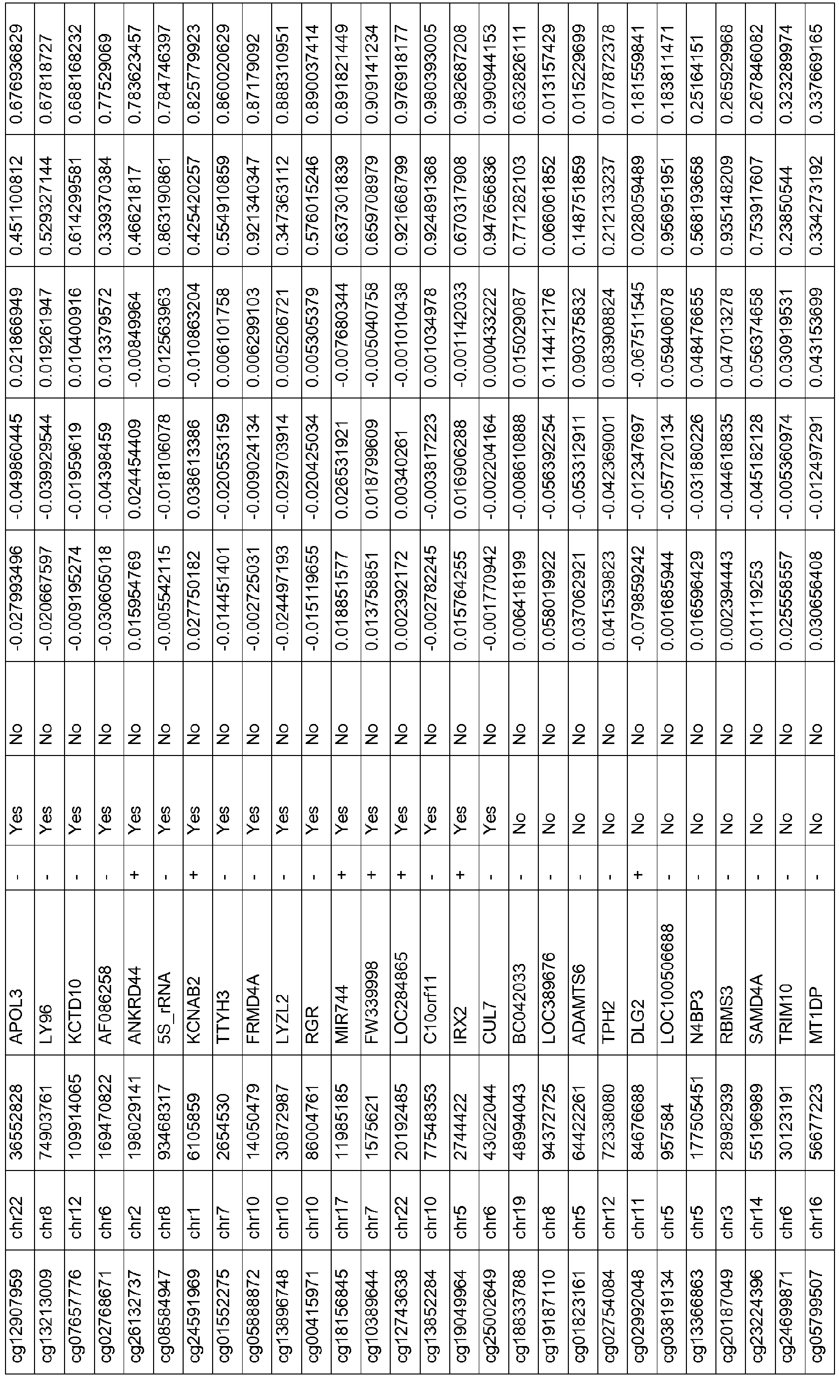

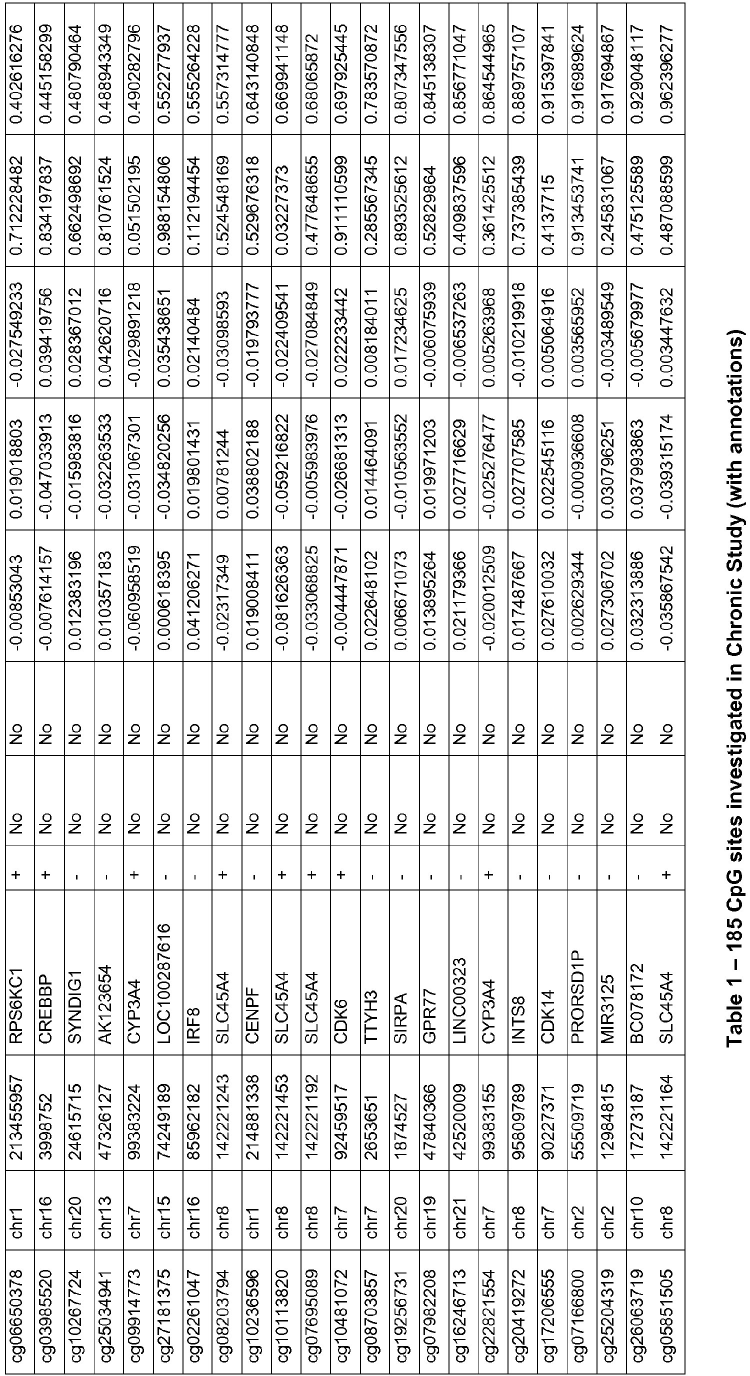

### Identification of CpG sites associated with use of sun protection products on chronic sun exposure

Of the 151 sites from the initial 185 that changed in the expected direction it can be seen that 3 of these sites also changed less in the treated group than the untreated group. Therefore these 3 CpG sites are valuable in the epigenetic evaluation of the use of sun protection products and their effect against chronic exposure because they are not as affected as those from the untreated arm. These sites are provided in Table 5.

cg02066936 cg16929393 cg05715751 cg21177821 cg05504117 cg00772091
cg06066180 cg00675160 cg00067720 cg02491003 cg24490133 cg05355684
cg19283806 cg23976431 cg00025972 cg07050101 cg10557907 cg25588389

cg15059474 cg08781365 cg08267250 cg16808156 cg13582226 cg02004370
cg06641366

The sites all have p-values calculated for the significance of the change in the untreated arm vs. the treated arm and therefore the sites can all be assessed for the significance of the probes in relation to the use of sun protection products.

Table 5 lists the 3 sites according to the invention with a p-value of at most 0.05.

Preferably all 3 sites of this subset are observed.

**Table 5**

| |
|---|
| cg02066936 cg16929393 cg06427571 |

### Assessment of CpG sites associated with use of sun protection products on acute sun exposure

A study was carried out to investigate the epigenetic effects of using sun protection products on acute sun exposure.

In the study, ex vivo human skin (discarded abdominal or breast skin from surgical procedures) was used.

Two aims of the study were:
- to evaluate the protective effects of the use of sun protection products against epigenetic modifications that may result from acute sun exposure, and
- to allow assessment of the ability of the 198 CpG sites identified above to measure the effects of the use of a sun protection product on acute sun exposure.

Three different treatments were employed:
1) Control (No UV exposure, no SPF)
   - On Day 0, fifteen 3mm punch biopsies were collected from ex vivo skin samples from 3 different donors.
   - The biopsies were placed into culture (5 biopsies per culture insert) to ensure viability for the experimental period.
   - Biopsy cultures were maintained for a further 7 days.
   - On day 7, Biopsies were snap frozen and stored at -80°C.
   - Genomic DNA was isolated (see below) within one week of snap freezing.
   - DNA Methylation changes were determined as detailed below.
2) UV Only (UV exposure, no SPF)
   - On Day 0 (a Tuesday), ex vivo skin samples from 3 different donors were subjected to UV irradiation (see below). Fifteen 3mm punch biopsies were collected from the skin samples following irradiation and placed into culture (5 biopsies per culture insert) to ensure viability for the experimental period.
   - Biopsy cultures were maintained for a further 7 days and subjected to UV irradiation (see below) daily (starting Wednesday, excluding weekend days).
   - After the last irradiation on day 7, biopsies were snap frozen and stored at -80°C.
   - Genomic DNA was isolated (see below) within one week of snap freezing.
   - DNA Methylation changes were determined as detailed below.
3) UV and SPF (UV exposure, with SPF applied)
   - On Day 0 (a Tuesday), 50uL of a product containing SPF (Pond's Age Miracle Firm and Lift Day Cream, Sun Protection Factor 30) was applied to a 3.8cm² area of ex vivo skin samples from 2 different donors. The skin samples were then subjected to UV irradiation (see below). Six 3mm punch biopsies were collected from the skin samples following irradiation and placed into culture (3 biopsies per culture insert) to ensure viability for the experimental period.
   - Biopsy cultures were maintained for a further 7 days and subjected to product application (2uL of product) and then UV irradiation (see below) daily (starting Wednesday, excluding weekend days).
   - After the last application/irradiation on day 7, biopsies were snap frozen and stored at -80°C.
   - Genomic DNA was isolated (see below) within one week of snap freezing.
   - DNA Methylation changes were determined as detailed below.

### METHOD FOR UV IRRADIATION

A Newport solar irradiator [Housing: 67005 / PSU: 69911 (160-600W) / Lamp: 6259 (300W Xe)] with CGA-320 and UG-11 filters to block UVC and visible light was used to dose the ex vivo skin or cultured biopsies with ssUV dose of equivalent to 1 MED (Minimal Erythema Dose). The 1MED dose was equivalent to 4J/cm2 (as detected by a UVA+UVB sensor from the Solar Light Company) and took 5 minutes and 40 seconds to deliver to the ex vivo skin or biopsies at a distance of -2.5cm.

### METHOD FOR ISOLATION OF GENOMIC DNA

Biopsies were removed from -80°C freezer. Adipose tissue was removed from the biopsy using surgical scissors. The remaining skin biopsy was digested and genomic DNA extracted following the protocol from the QiaAMP DNA kit (Qiagen). The gDNA was resuspended in 1x Tris-EDTA (1x TE) and stored at -20C until use.

### METHOD FOR DETERMINATION OF METHYLATION CHANGES

Isolated genomic DNA was sent to Q Squared Solutions Expression Analysis LLC to perform their methylation analysis using their Illumina Infinium DNA Methylation Service and the Illumina MethylationEPIC BeadChips. Methylation analysis included:
- performing bisulfite conversion of genomic DNA
- amplifying genomic DNA using whole genome techniques
- hybridizing amplified DNA to Infinium BeadChip
- performing single base extension reactions with labeled allele-specific, dideoxynucleotides
- staining and scanning of the BeadChip
- performing DNA Methylation analysis and providing results

### EPIGENETIC ANALYSIS

Of the 198 CpG sites identified above that were therefore taken forward and assessed in the context of acute sun exposure and sun protection, 185 sites were present on the MethylationEPIC BeadChip arrays used.

Samples were filtered to remove probes that contained known polymorphisms (23,399 probes), were cross-hybridised (42,557 probes) and non-CpG probes (1,651 probes) identified by McCartney et al, 2016. Those probes on X and Y chromosomes and those probes where more than 25% of samples had a signal indistinguishable from negative control probes were also removed (3,682 probes). From a total of 866,836, 80,683 probes were excluded from the raw data, leaving 786,153 probes. None of the above 185 sites/probes were lost as a result of this filtering.

The aim of the epigenetic analysis was to:
- Identify the extent of methylation on the 185 CpG sites as a result of acute UV exposure. This was done by comparing DNAm in Control samples (No UV exposure, no SPF) against those subjected to UV Only (UV exposure, no SPF). This is referred to as "**UV vs. Control**".
- The aim was also to identify if methylation on the same 185 CpG sites was different as a result of using SPF products to protect against acute UV exposure by comparing DNAm in Control samples (No UV exposure, no SPF) against those subjected to UV but with SPF applied (UV exposure, with SPF applied). This is referred to as **"UV+SPF vs. Control**".
- The results of "UV vs. Control" was then compared against "UV+SPF vs. Control"

### RESULTS

For each probe and sample type, the mean absolute change in M-value (from baseline) was calculated over samples. It was then determined whether this change was different in "UV vs. Control" compared to "UV+SPF vs. Control" over the set of probes.

To visualise changes for each of the 185 probes (averaged over samples within a treatment group), box plots (Figure 3) were generated for the "UV vs. Control" changes (right hand box plot) and the "UV+SPF vs. Control" (left hand box plot) changes separately. Each point in the boxplot shows the mean M-value measure (mean absolute change in M-value from baseline over samples) for a given probe and treatment group. One-way analysis of these changes shows a significance (T-test) of P=0.0008. Therefore, from Figure 3 it can be seen that as a consequence of SPF use, the epigenetic changes were reduced relative to the non-SPF samples.

It is therefore apparent that selections of these 185 CpG sites, either alone or in combination, are capable of measuring epigenetic changes associated with or caused by acute sun exposure. In addition, these CpG sites are capable of measuring the beneficial effects of using sun protection products to prevent the epigenetic effects of acute sun exposure.

It is remarkable that methylation changes were observed in this specifically selected (predefined) probe set. Moreover, it is a novel insight that epigenetic changes were observed over just 1 week - typically such changes have been associated with many years of exposure.

The 185 sites are shown in Table 6 in which annotations are based on Human Genome Build HG19 and the columns are:
- CpG site/loci ID
- Chromosome
- Probe start position
- Closest gene name
- Expected change in CpG site over time (where '+' means hyper-methylated, '-' means hypo- methylated)
- Whether CpG site was found to change in the expected direction (Yes/No)
- Whether CpG site was found to change in the expected direction AND showed greater change in the untreated sample than the treated sample (Yes/No)
- deltaM untreated (change in M-values of untreated sample)
- deltaM treated (change in M-values of treated sample)
- Difference in deltaM between untreated and treated samples
- P-value for change in untreated sample
- P-value for change in untreated vs. treated sample

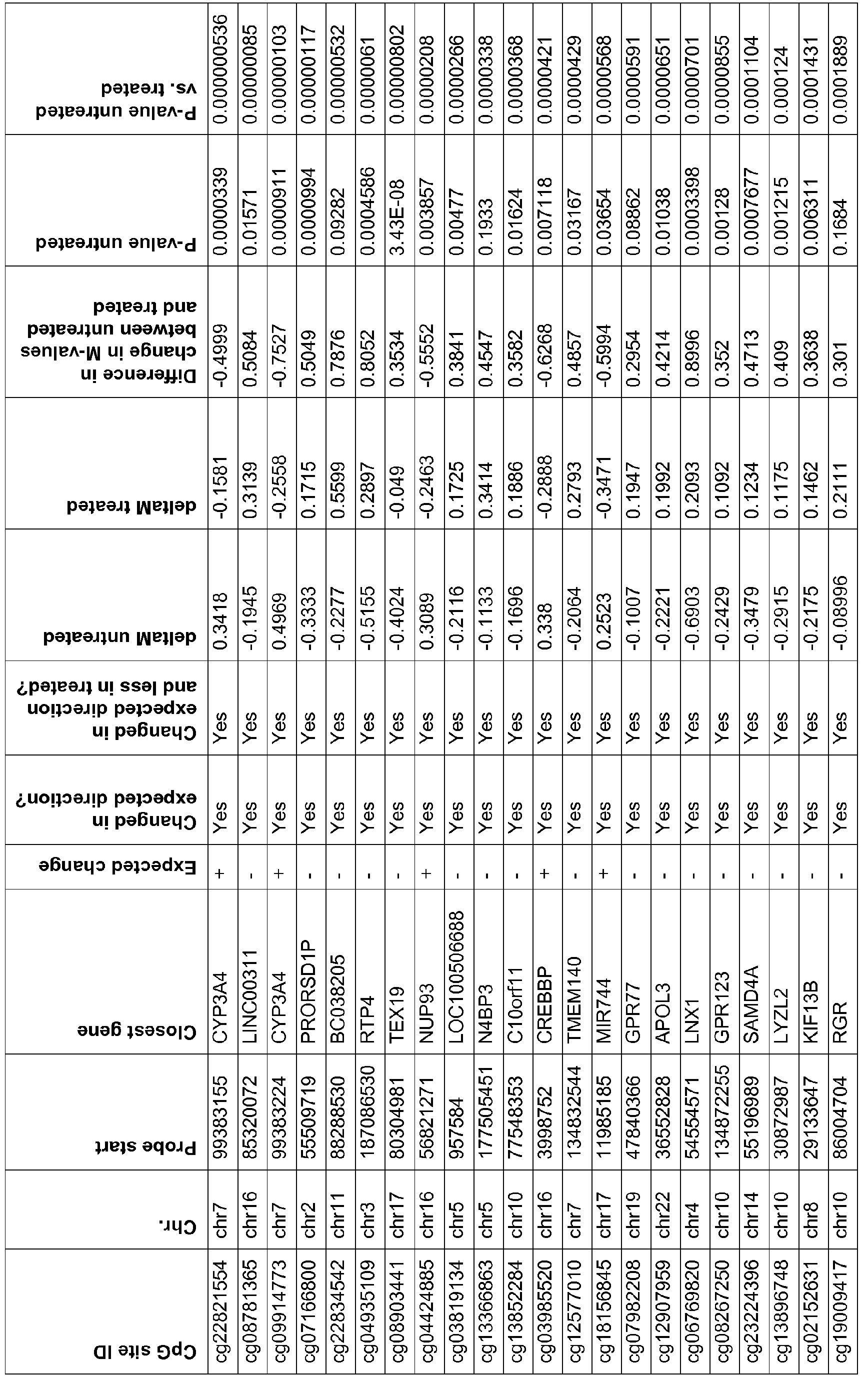

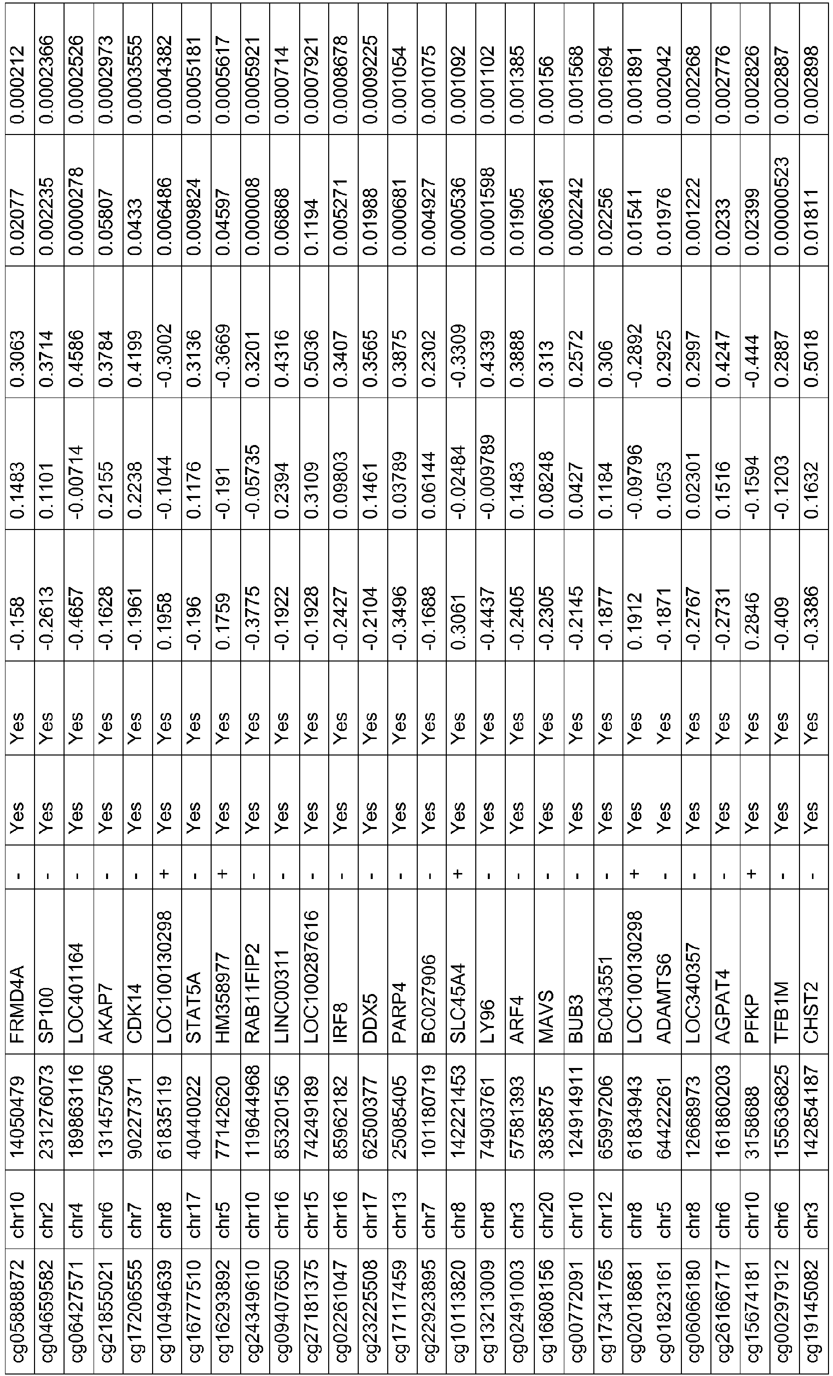

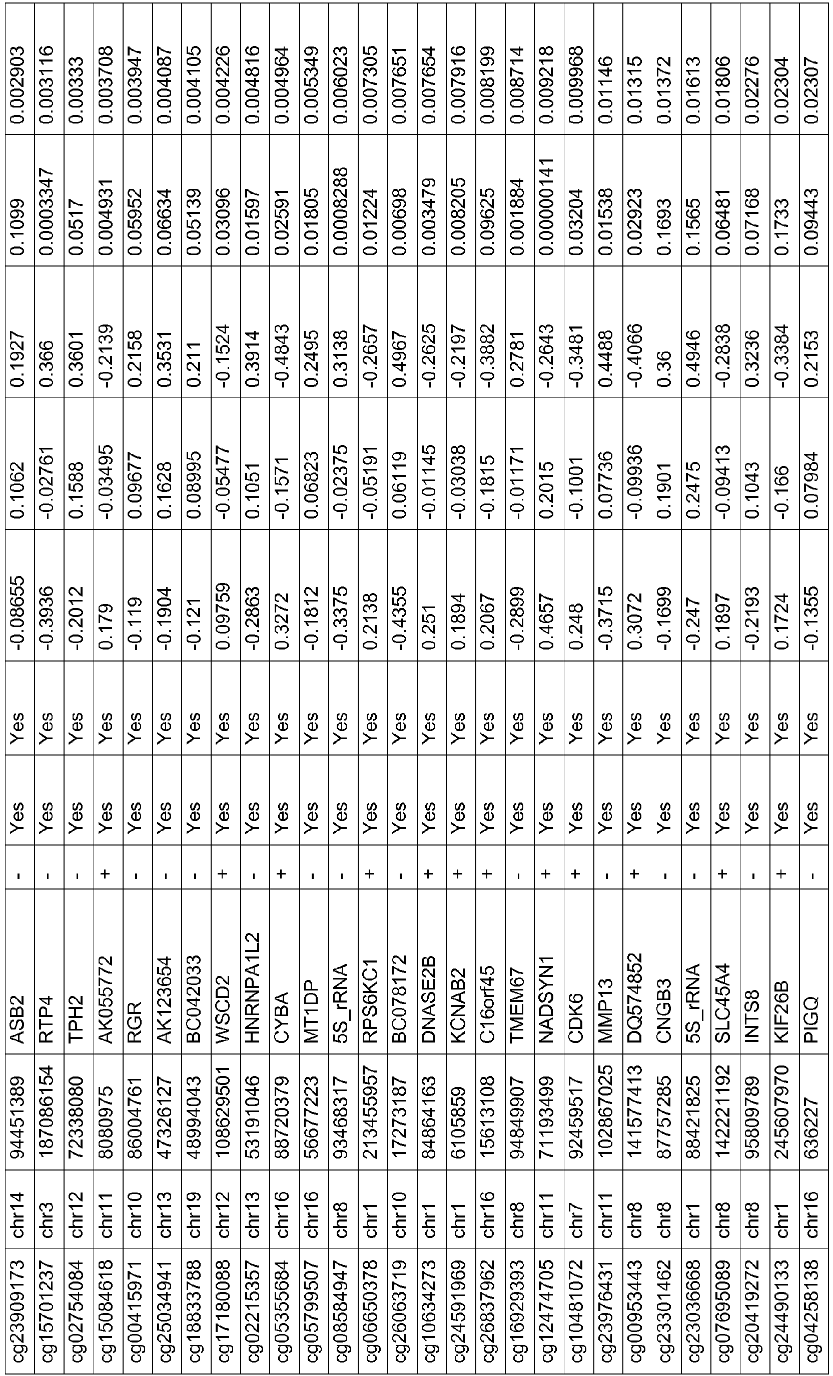

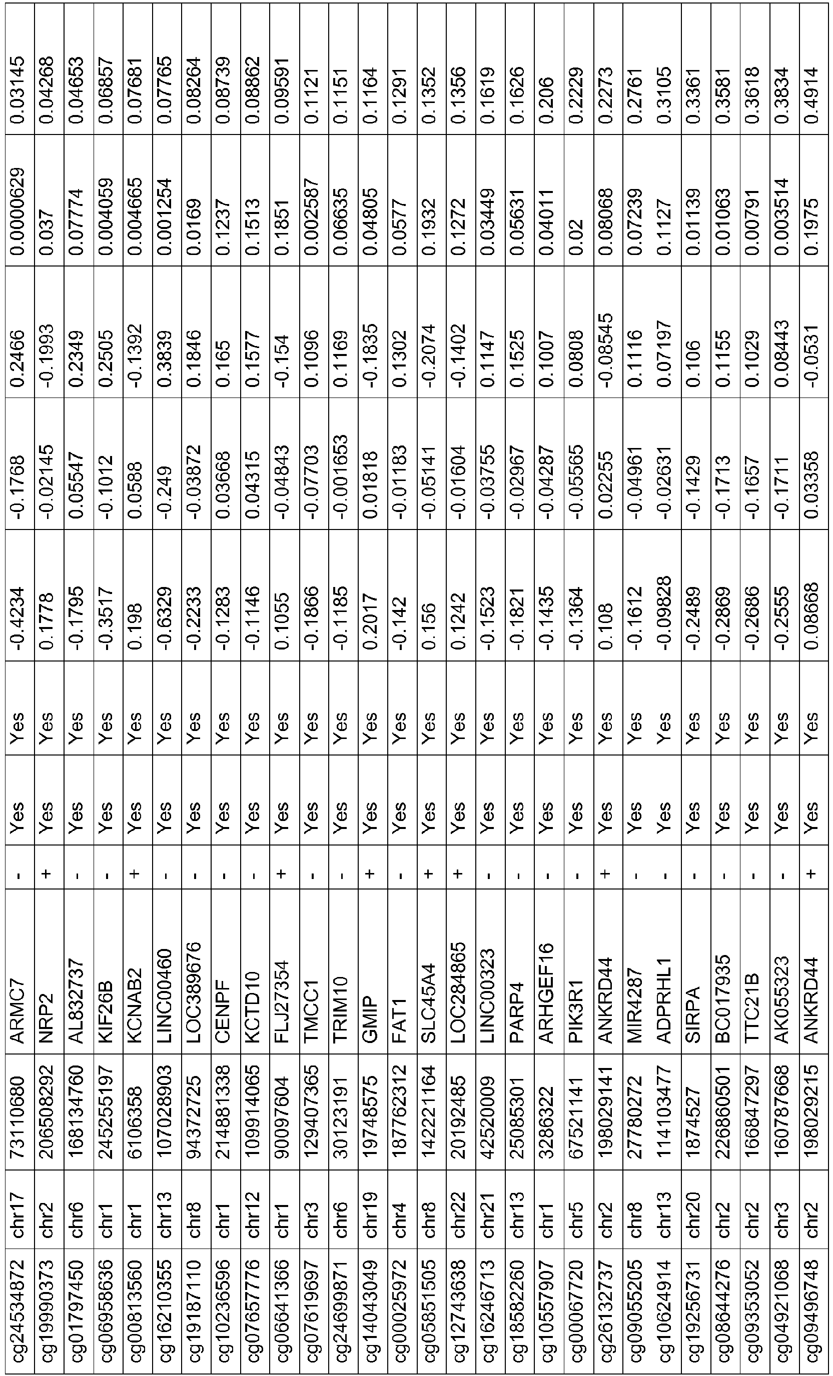

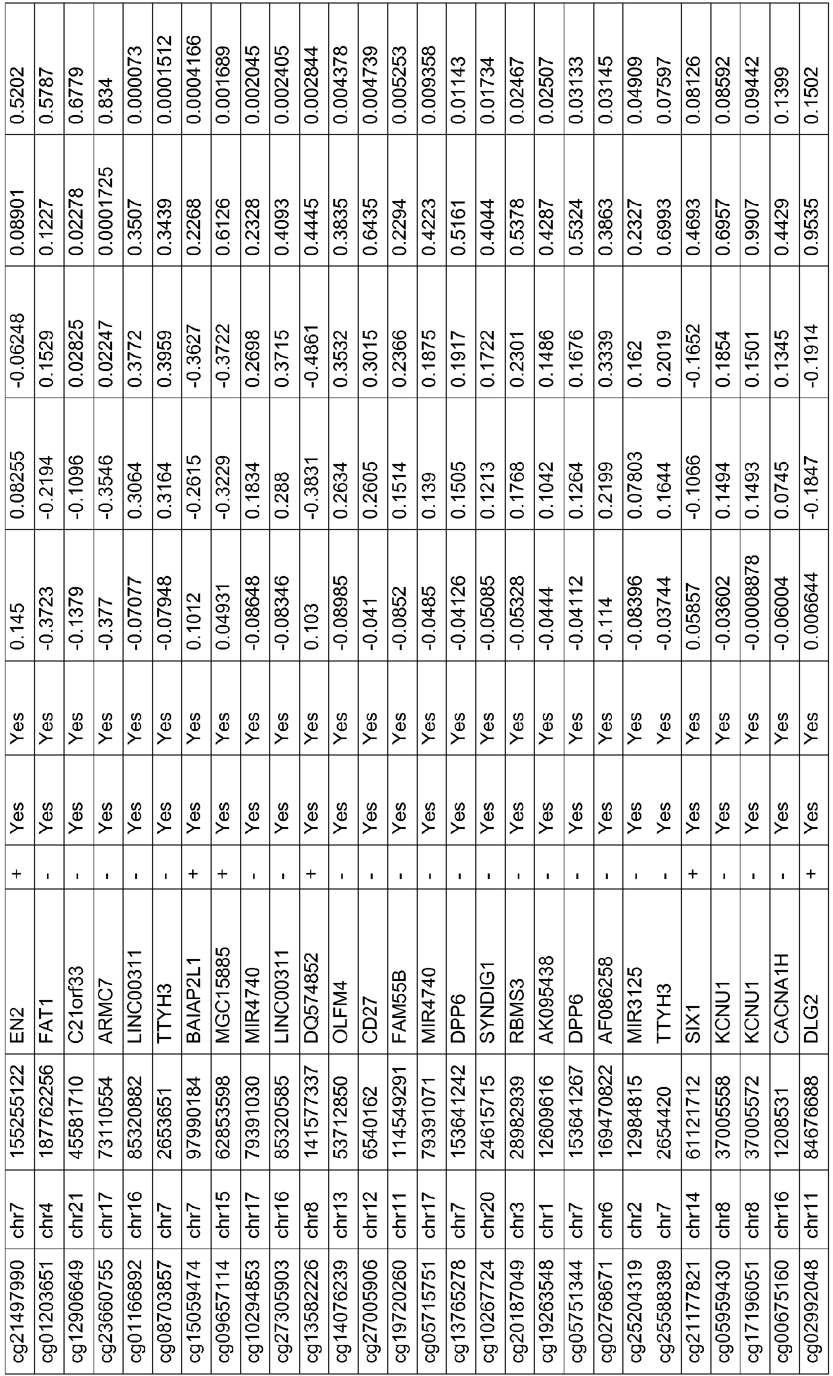

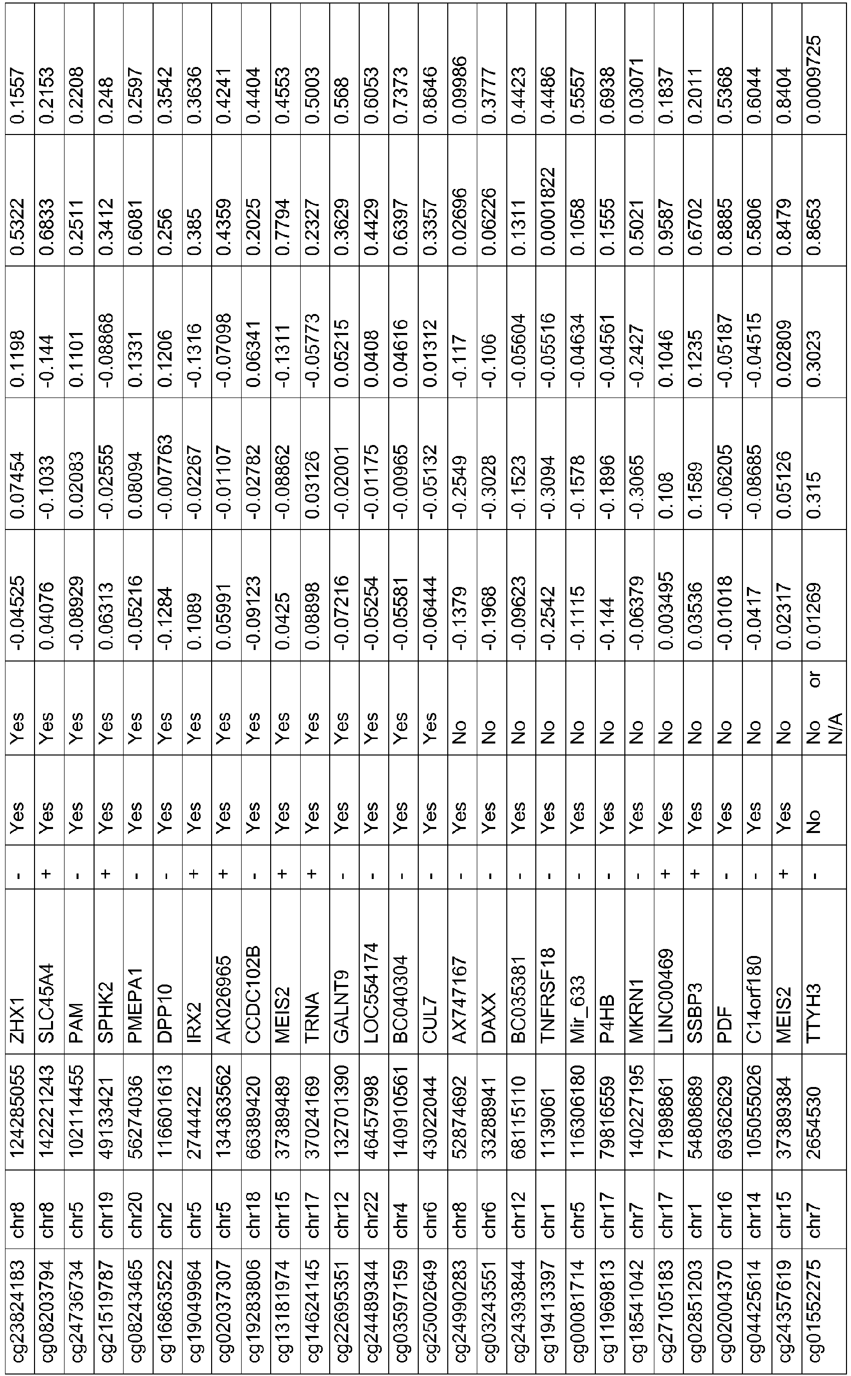

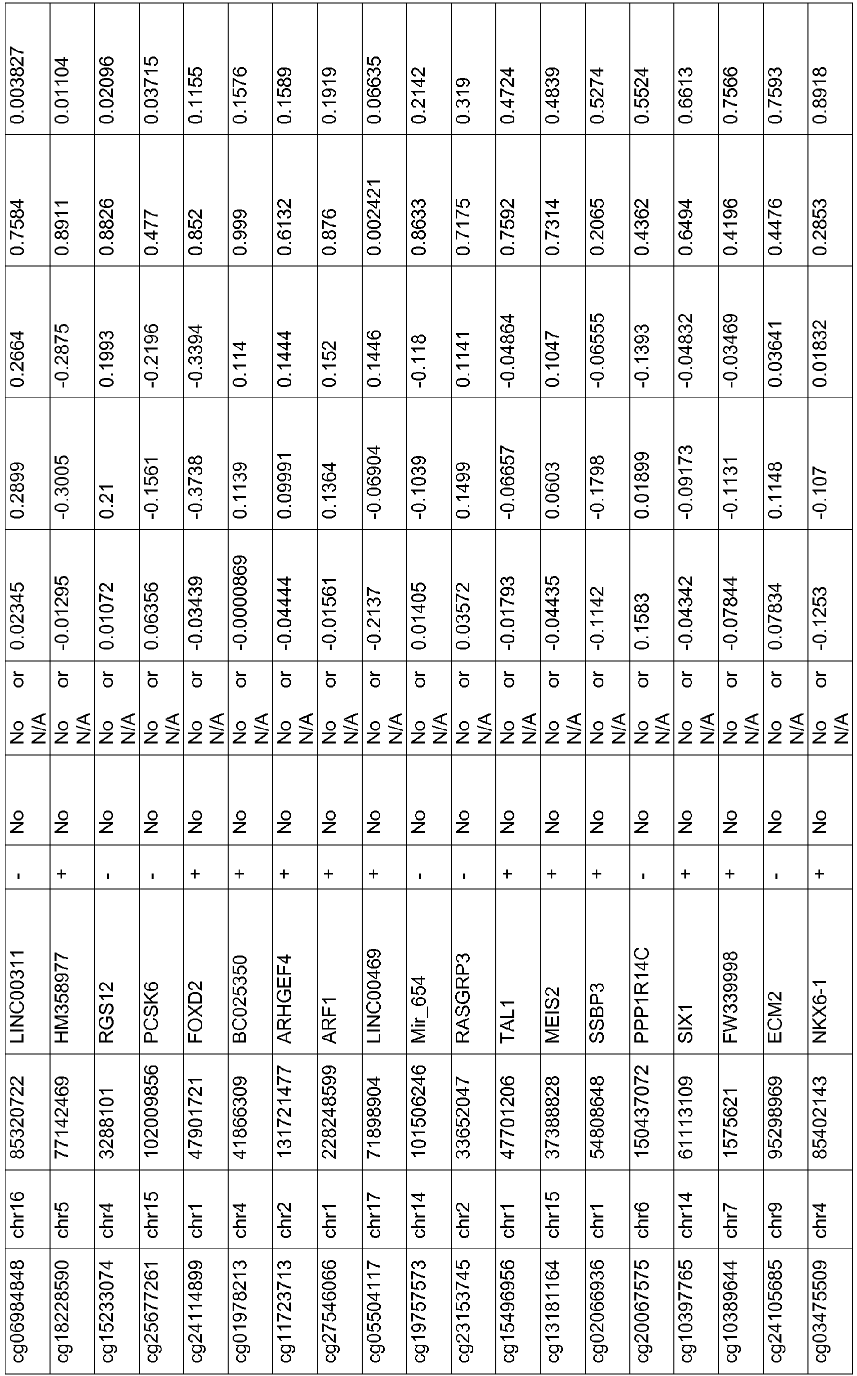

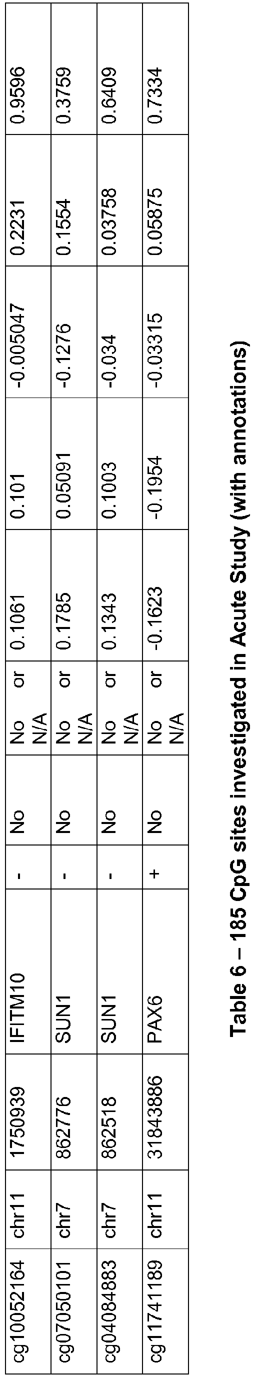

It can be seen from Table 6 that there were 161 sites from the initial 185 that changed in the expected direction (hyper- or hypo-methylated). Of these 161 sites from the initial 185 that changed in the expected direction, 149 sites also change less in the treated group than the untreated group. Therefore these 149 are indicative of protection against acute exposure because they are not as affected as those from the untreated sample. These sites are provided in Table 7.

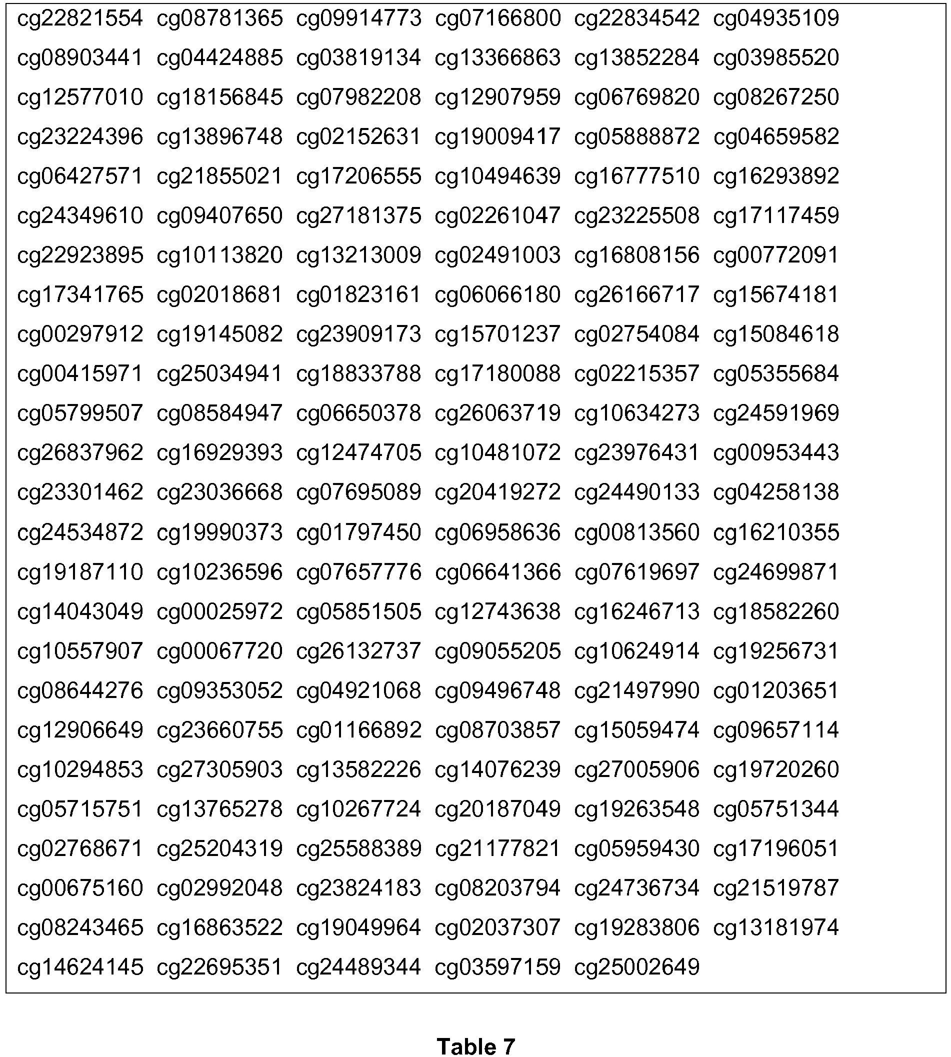

These 149 sites therefore have greater performance for the epigenetic assessment of use of sun protection products on acute sun exposure.

The sites all have p-values calculated for the significance of the change in the untreated sample vs. the treated sample and therefore the sites can all be assessed for the significance of the probes in relation to the use of sun protection.

cg22821554 cg08781365 cg09914773 cg07166800 cg22834542 cg04935109

Table 10 lists the 99 sites according to the invention with p-value of at most 0.05.

Preferably >5, >10, >15, >20, >30, >50, >75, >90, most preferably all 99 sites of this subset are observed.

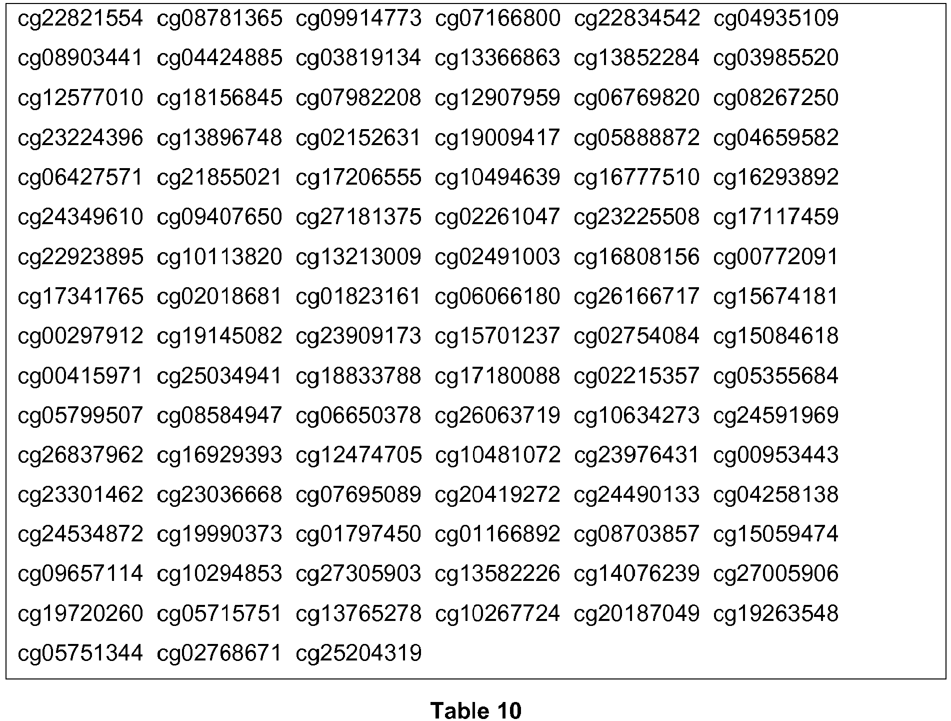

Table 11 lists the 81 sites with a p-value of at most 0.01. These sites are a still more preferable subset.

Preferably >5, >10, >15, >20, >30, >50, >75, most preferably all 81 sites of this subset are observed.

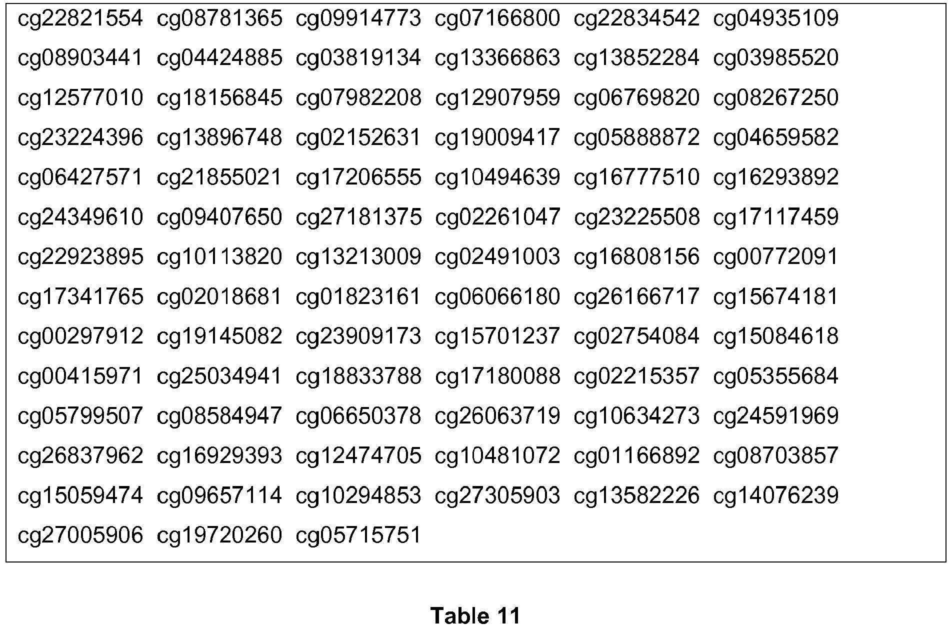

Table 12 lists the 38 sites with a p-value of at most 0.001. These sites are the most preferable subset.

Preferably >5, >10, >15, >20, >30, >35, most preferably all 38 sites of this subset are observed.

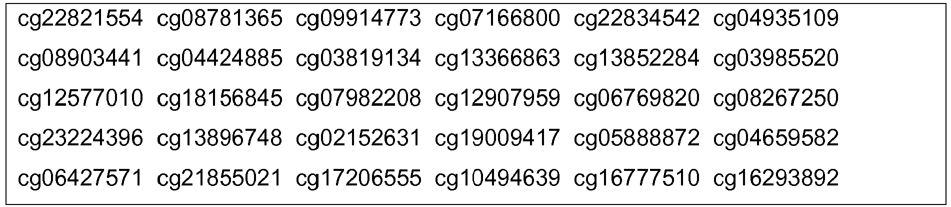

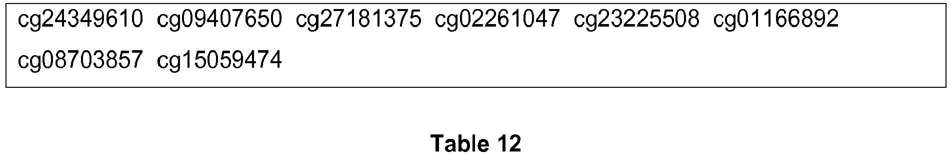

## Claims

1. A method for obtaining information useful to determine the effect of the use of sun protection products on chronic sun exposure on the skin of an individual, the method comprising the steps of:
(a) obtaining genomic DNA from skin cells derived from the individual, wherein the skin cells include epidermal skin cells; and
(b) observing cytosine methylation of at least two CpG loci in the genomic DNA selected from the group consisting of:
cg02066936 cg16929393 cg06427571,
so that information useful to determine the effect of the use of sun protection products on chronic sun exposure is obtained.

2. A method for obtaining information useful to determine the effect of the use of sun protection products on acute sun exposure on the skin of an individual, the method comprising the steps of:
(a) obtaining genomic DNA from skin cells derived from the individual, wherein the skin cells include epidermal skin cells; and
(b) observing cytosine methylation of at least two CpG loci in the genomic DNA selected from the group consisting of: 
cg22821554 cg08781365 cg09914773 cg07166800 cg22834542 cg04935109
cg08903441 cg04424885 cg03819134 cg13366863 cg13852284 cg03985520
cg12577010 cg18156845 cg07982208 cg12907959 cg06769820 cg08267250
cg23224396 cg13896748 cg02152631 cg19009417 cg05888872 cg04659582
cg06427571 cg21855021 cg17206555 cg10494639 cg16777510 cg16293892
cg24349610 cg09407650 cg27181375 cg02261047 cg23225508 cg17117459
cg22923895 cg10113820 cg13213009 cg02491003 cg16808156 cg00772091
cg17341765 cg02018681 cg01823161 cg06066180 cg26166717 cg15674181
cg00297912 cg19145082 cg23909173 cg15701237 cg02754084 cg15084618
cg00415971 cg25034941 cg18833788 cg17180088 cg02215357 cg05355684
cg05799507 cg08584947 cg06650378 cg26063719 cg10634273 cg24591969
cg26837962 cg16929393 cg12474705 cg10481072 cg23976431 cg00953443
cg23301462 cg23036668 cg07695089 cg20419272 cg24490133 cg04258138
 
cg24534872 cg19990373 cg01797450 cg01166892 cg08703857 cg15059474
cg09657114 cg10294853 cg27305903 cg13582226 cg14076239 cg27005906
cg19720260 cg05715751 cg13765278 cg10267724 cg20187049 cg19263548
cg05751344 cg02768671 cg25204319,
 so that information useful to determine the effect of the use of sun protection products on acute sun exposure is obtained.

3. . A method according to claim 2 for obtaining information useful to determine the effect of the use of sun protection products on acute sun exposure on the skin of an individual, the method comprising the steps of:
(a) obtaining genomic DNA from skin cells derived from the individual, wherein the skin cells include epidermal skin cells; and
(b) observing cytosine methylation of at least two CpG loci in the genomic DNA selected from the group consisting of: 
cg22821554 cg08781365 cg09914773 cg07166800 cg22834542 cg04935109
cg08903441 cg04424885 cg03819134 cg13366863 cg13852284 cg03985520
cg12577010 cg18156845 cg07982208 cg12907959 cg06769820 cg08267250
cg23224396 cg13896748 cg02152631 cg19009417 cg05888872 cg04659582
cg06427571 cg21855021 cg17206555 cg10494639 cg16777510 cg16293892
cg24349610 cg09407650 cg27181375 cg02261047 cg23225508 cg17117459
cg22923895 cg10113820 cg13213009 cg02491003 cg16808156 cg00772091
cg17341765 cg02018681 cg01823161 cg06066180 cg26166717 cg15674181
cg00297912 cg19145082 cg23909173 cg15701237 cg02754084 cg15084618
cg00415971 cg25034941 cg18833788 cg17180088 cg02215357 cg05355684
cg05799507 cg08584947 cg06650378 cg26063719 cg10634273 cg24591969
cg26837962 cg16929393 cg12474705 cg10481072 cg01166892 cg08703857
cg15059474 cg09657114 cg10294853 cg27305903 cg13582226 cg14076239
cg27005906 cg19720260 cg05715751,
 so that information useful to determine the effect of the use of sun protection products on acute sun exposure is obtained.

4. A method according to claim 3 for obtaining information useful to determine the effect of the use of sun protection products on acute sun exposure on the skin of an individual, the method comprising the steps of:
(a) obtaining genomic DNA from skin cells derived from the individual, wherein the skin cells include epidermal skin cells; and
(b) observing cytosine methylation of at least two CpG loci in the genomic DNA selected from the group consisting of: 
cg22821554 cg08781365 cg09914773 cg07166800 cg22834542 cg04935109
cg08903441 cg04424885 cg03819134 cg13366863 cg13852284 cg03985520
cg12577010 cg18156845 cg07982208 cg12907959 cg06769820 cg08267250
cg23224396 cg13896748 cg02152631 cg19009417 cg05888872 cg04659582
cg06427571 cg21855021 cg17206555 cg10494639 cg16777510 cg16293892
cg24349610 cg09407650 cg27181375 cg02261047 cg23225508 cg01166892
cg08703857 cg15059474,
 so that information useful to determine the effect of the use of sun protection products on acute sun exposure is obtained.

## Patentansprüche

1. Verfahren zum Erhalt von Informationen, die nützlich sind, um die Wirkung der Verwendung von Sonnenschutzprodukten bei chronischer Sonnenexposition auf der Haut eines Individuums zu bestimmen, wobei das Verfahren die Schritte umfasst:
(a) Erhalten genomischer DNA aus Hautzellen, die von dem Individuum stammen, wobei die Hautzellen epidermale Hautzellen umfassen; und
(b) Beobachten der Cytosin-Methylierung von mindestens zwei CpG-Stellen in der genomischen DNA, ausgewählt aus der Gruppe, bestehend aus:
cg02066936 cg16929393 cg06427571,
damit die Informationen, die nützlich sind, um die Wirkung der Verwendung von Sonnenschutzprodukten bei chronischer Sonnenexposition zu bestimmen, erhalten werden.

2. Verfahren zum Erhalt von Informationen, die nützlich sind, um die Wirkung der Verwendung von Sonnenschutzprodukten bei akuter Sonnenexposition auf der Haut eines Individuums zu bestimmen, wobei das Verfahren die Schritte umfasst:
(a) Erhalten genomischer DNA aus Hautzellen, die von dem Individuum stammen, wobei die Hautzellen epidermale Hautzellen umfassen; und
(b) Beobachten der Cytosin-Methylierung von mindestens zwei CpG-Stellen in der genomischen DNA, ausgewählt aus der Gruppe, bestehend aus: 
cg22821554 cg08781365 cg09914773 cg07166800 cg22834542 cg04935109
cg08903441 cg04424885 cg03819134 cg13366863 cg13852284 cg03985520
cg12577010 cg18156845 cg07982208 cg12907959 cg06769820 cg08267250
cg23224396 cg13896748 cg02152631 cg19009417 cg05888872 cg04659582
cg06427571 cg21855021 cg17206555 cg10494639 cg16777510 cg16293892
cg24349610 cg09407650 cg27181375 cg02261047 cg23225508 cg17117459
cg22923895 cg10113820 cg13213009 cg02491003 cg16808156 cg00772091
cg17341765 cg02018681 cg01823161 cg06066180 cg26166717 cg15674181
cg00297912 cg19145082 cg23909173 cg15701237 cg02754084 cg15084618
cg00415971 cg25034941 cg18833788 cg17180088 cg02215357 cg05355684
cg05799507 cg08584947 cg06650378 cg26063719 cg10634273 cg24591969
cg26837962 cg16929393 cg12474705 cg10481072 cg23976431 cg00953443
cg23301462 cg23036668 cg07695089 cg20419272 cg24490133 cg04258138
cg24534872 cg19990373 cg01797450 cg01166892 cg08703857 cg15059474
cg09657114 cg10294853 cg27305903 cg13582226 cg14076239 cg27005906
cg19720260 cg05715751 cg13765278 cg10267724 cg20187049 cg19263548
cg05751344 cg02768671 cg25204319,
 damit die Informationen, die nützlich sind, um die Wirkung der Verwendung von Sonnenschutzprodukten bei akuter Sonnenexposition zu bestimmen, erhalten werden.

3. Verfahren nach Anspruch 2, um Informationen zu erhalten, die nützlich sind, um die Wirkung der Verwendung von Sonnenschutzprodukten bei akuter Sonnenexposition auf der Haut eines Individuums zu bestimmen, wobei das Verfahren die Schritte umfasst:
(a) Erhalten genomischer DNA aus Hautzellen, die von dem Individuum stammen, wobei die Hautzellen epidermale Hautzellen umfassen; und
(b) Beobachten der Cytosin-Methylierung von mindestens zwei CpG-Stellen in der genomischen DNA, ausgewählt aus der Gruppe, bestehend aus: 
cg22821554 cg08781365 cg09914773 cg07166800 cg22834542 cg04935109
cg08903441 cg04424885 cg03819134 cg13366863 cg13852284 cg03985520
cg12577010 cg18156845 cg07982208 cg12907959 cg06769820 cg08267250
cg23224396 cg13896748 cg02152631 cg19009417 cg05888872 cg04659582
cg06427571 cg21855021 cg17206555 cg10494639 cg16777510 cg16293892
cg24349610 cg09407650 cg27181375 cg02261047 cg23225508 cg17117459
cg22923895 cg10113820 cg13213009 cg02491003 cg16808156 cg00772091
cg17341765 cg02018681 cg01823161 cg06066180 cg26166717 cg15674181
cg00297912 cg19145082 cg23909173 cg15701237 cg02754084 cg15084618
cg00415971 cg25034941 cg18833788 cg17180088 cg02215357 cg05355684
cg05799507 cg08584947 cg06650378 cg26063719 cg10634273 cg24591969
cg26837962 cg16929393 cg12474705 cg10481072 cg01166892 cg08703857
cg15059474 cg09657114 cg10294853 cg27305903 cg13582226 cg14076239
cg27005906 cg19720260 cg05715751,
 damit die Informationen, die nützlich sind, um die Wirkung der Verwendung von Sonnenschutzprodukten bei akuter Sonnenexposition zu bestimmen, erhalten werden.

4. Verfahren nach Anspruch 3, um Informationen zu erhalten, die nützlich sind, um die Wirkung der Verwendung von Sonnenschutzprodukten bei akuter Sonnenexposition auf der Haut eines Individuums zu bestimmen, wobei das Verfahren die Schritte umfasst:
(a) Erhalten genomischer DNA aus Hautzellen, die von dem Individuum stammen, wobei die Hautzellen epidermale Hautzellen umfassen; und
(b) Beobachten der Cytosin-Methylierung von mindestens zwei CpG-Stellen in der genomischen DNA, ausgewählt aus der Gruppe, bestehend aus: 
cg22821554 cg08781365 cg09914773 cg07166800 cg22834542 cg04935109
cg08903441 cg04424885 cg03819134 cg13366863 cg13852284 cg03985520
cg12577010 cg18156845 cg07982208 cg12907959 cg06769820 cg08267250
cg23224396 cg13896748 cg02152631 cg19009417 cg05888872 cg04659582
cg06427571 cg21855021 cg17206555 cg10494639 cg16777510 cg16293892
cg24349610 cg09407650 cg27181375 cg02261047 cg23225508 cg01166892
cg08703857 cg15059474,
 damit die Informationen, die nützlich sind, um die Wirkung der Verwendung von Sonnenschutzprodukten bei akuter Sonnenexposition zu bestimmen, erhalten werden.

## Revendications

1. Procédé pour l'obtention d'information utile pour déterminer l'effet de l'utilisation de produits de protection solaire sur une exposition chronique au soleil sur la peau d'un individu, le procédé comprenant les étapes de :
(a) obtention d'ADN génomique à partir de cellules cutanées dérivées de l'individu, dans lequel les cellules cutanées incluent des cellules cutanées de l'épiderme ; et
(b) observation de la méthylation de la cytosine d'au moins deux locus CpG dans l'ADN génomique choisis dans le groupe consistant en :
cg02066936 cg16929393 cg 06427571,
de sorte qu'une information utile pour déterminer l'effet de l'utilisation de produits de protection solaire sur l'exposition chronique au soleil est obtenue.

2. Procédé pour l'obtention d'information utile pour déterminer l'effet de l'utilisation de produits de protection solaire sur une exposition intense au soleil de la peau d'un individu, le procédé comprenant les étapes de :
(a) obtention d'ADN génomique à partir de cellules cutanées dérivées de l'individu, dans lequel les cellules cutanées incluent des cellules cutanées de l'épiderme ; et
(b) observation de la méthylation de la cytosine d'au moins deux locus CpG dans l'ADN génomique choisis dans le groupe consistant en : 
cg22821554 cg08781365 cg09914773 cg07166800 cg22834542 cg04935109
cg08903441 cg04424885 cg03819134 cg13366863 cg13852284 cg03985520
cg12577010 cg18156845 cg07982208 cg12907959 cg06769820 cg08267250
cg23224396 cg13896748 cg02152631 cg19009417 cg05888872 cg04659582
cg06427571 cg21855021 cg17206555 cg10494639 cg16777510 cg16293892
cg24349610 cg09407650 cg27181375 cg02261047 cg23225508 cg17117459
cg22923895 cg10113820 cg13213009 cg02491003 cg16808156 cg00772091
cg17341765 cg02018681 cg01823161 cg06066180 cg26166717 cg15674181
cg00297912 cg19145082 cg23909173 cg15701237 cg02754084 cg15084618
cg00415971 cg25034941 cg18833788 cg17180088 cg02215357 cg05355684
 
cg05799507 cg08584947 cg06650378 cg26063719 cg10634273 cg24591969
cg26837962 cg16929393 cg12474705 cg10481072 cg23976431 cg00953443
cg23301462 cg23036668 cg07695089 cg20419272 cg24490133 cg04258138
cg24534872 cg19990373 cg01797450 cg01166892 cg08703857 cg15059474
cg09657114 cg10294853 cg27305903 cg13582226 cg14076239 cg27005906
cg19720260 cg05715751 cg13765278 cg10267724 cg20187049 cg19263548
cg05751344 cg02768671 cg25204319,
 de sorte qu'une information utile pour déterminer l'effet de l'utilisation de produits de protection solaire sur une exposition intense au soleil est obtenue.

3. Procédé selon la revendication 2 pour l'obtention d'information utile pour déterminer l'effet de l'utilisation de produits pour la protection solaire sur une exposition intense au soleil sur la peau d'un individu, le procédé comprenant les étapes de :
(a) obtention d'ADN génomique à partir de cellules cutanées dérivées de l'individu, dans lequel les cellules cutanées incluent des cellules cutanées de l'épiderme ; et
(b) observation de la méthylation de la cytosine d'au moins deux locus CpG dans l'ADN génomique choisis dans le groupe consistant en : 
cg22821554 cg08781365 cg09914773 cg07166800 cg22834542 cg04935109
cg08903441 cg04424885 cg03819134 cg13366863 cg13852284 cg03985520
cg12577010 cg18156845 cg07982208 cg12907959 cg06769820 cg08267250
cg23224396 cg13896748 cg02152631 cg19009417 cg05888872 cg04659582
cg06427571 cg21855021 cg17206555 cg10494639 cg16777510 cg16293892
cg24349610 cg09407650 cg27181375 cg02261047 cg23225508 cg17117459
cg22923895 cg10113820 cg13213009 cg02491003 cg16808156 cg00772091
cg17341765 cg02018681 cg01823161 cg06066180 cg26166717 cg15674181
cg00297912 cg19145082 cg23909173 cg15701237 cg02754084 cg15084618
cg00415971 cg25034941 cg18833788 cg17180088 cg02215357 cg05355684
cg05799507 cg08584947 cg06650378 cg26063719 cg10634273 cg24591969
cg26837962 cg16929393 cg12474705 cg10481072 cg01166892 cg08703857
cg15059474 cg09657114 cg10294853 cg27305903 cg13582226 cg14076239
cg27005906 cg19720260 cg05715751,
 de sorte qu'une information utile pour déterminer l'effet de l'utilisation de produits de protection solaire sur une exposition intense au soleil est obtenue.

4. Procédé selon la revendication 3 pour l'obtention d'information utile pour déterminer l'effet de l'utilisation de produits de protection solaire sur l'exposition intense au soleil sur la peau d'un individu, le procédé comprenant les étapes de :
(a) obtention d'ADN génomique à partir de cellules cutanées dérivées de l'individu, dans lequel les cellules cutanées incluent des cellules cutanées de l'épiderme ; et
(b) observation de la méthylation de la cytosine d'au moins deux locus CpG dans l'ADN génomique choisis dans le groupe consistant en : 
cg22821554 cg08781365 cg09914773 cg07166800 cg22834542 cg04935109
cg08903441 cg04424885 cg03819134 cg13366863 cg13852284 cg03985520
cg12577010 cg18156845 cg07982208 cg12907959 cg06769820 cg08267250
cg23224396 cg13896748 cg02152631 cg19009417 cg05888872 cg04659582
cg06427571 cg21855021 cg17206555 cg10494639 cg16777510 cg16293892
cg24349610 cg09407650 cg27181375 cg02261047 cg23225508 cg01166892
cg08703857 cg15059474,
 de sorte qu'une information utile pour déterminer l'effet de l'utilisation de produits de protection solaire sur une exposition intense au soleil est obtenue.
